# EUROPEAN PATENT APPLICATION

(11) **EP 2 618 147 A1**
(43) Date of publication of application: **24.07.2013**
(21) Application number: 11818262.5
(22) Date of filing: 15.08.2011
(51) Int. Cl.: G01N 33/53, A01K 67/027, A61K 35/00, A61K 35/28, A61K 39/395, A61K 45/00, A61K 48/00, C12Q 1/68, G01N 33/574, C12N 15/09, C12N 15/113

(54) **REAGENT FOR TUMOR TESTING AND PHARMACEUTICAL COMPOSITION FOR TUMOR PREVENTION**

(30) Priority: 16.08.2010 JP 2010181835
(71) Applicant: Kyushu University, National University Corporation, Fukuoka-shi Fukuoka 812-8581 (JP)
(72) Inventor: TAKAHASHI, Atsushi, Fukuoka-shi, Fukuoka 812-8581 (JP)
(74) Representative: Woods, Geoffrey Corlett
(86) International application number: PCT/JP2011/068814
(87) International publication number: WO 2012/023622

(57) **Abstract**

The present invention aims to provide a novel reagent for tumor testing and a novel pharmaceutical composition for tumor prevention.

The present invention provides a reagent for tumor detection, which comprises a probe for the FEAT gene or amplification primers for the FEAT gene, or an antibody against the FEAT protein or a fragment of the antibody. Moreover, the present invention also provides a pharmaceutical composition for tumor prevention, which is configured to use the FEAT gene or the FEAT protein as a tumor marker to thereby recognize tumor cells.

## Description

### TECHNICAL FIELD

The present invention relates to a reagent for tumor detection and a pharmaceutical composition for tumor prevention, each of which comprises a probe for the FEAT gene or amplification primers for the FEAT gene, or an antibody against the FEAT protein or a fragment of the antibody.

### BACKGROUND ART

Although progresses have been made in elucidating the molecular biological mechanisms of carcinogenesis, there has not been established any method effective for cancer screening and cancer prevention (Non-patent Document 1). Thus, to establish a method for molecular-targeted cancer screening and prevention, it would be necessary to explore, characterize and classify cancer genes involved in diverse cancers.

However, as a result of attempts to comprehensively identify cancer genes by using procedures such as comprehensive sequencing of protein-coding exon regions (Non-patent Documents 2 to 4), whole genome sequencing (Non-patent Document 5), and paired-end sequencing for identification of gene rearrangements in somatic cells (Non-patent Document 6), it has been found that the processes of tumorigenesis in humans are markedly diverse and complex; and nobody has succeeded yet in finding out any feature common to various types of cancers. Gene mutations contributing to the development of human cancers are highly variable among different types of cancers and among individual tumor tissues even in the same type of cancer (Non-patent Document 7). For this reason, it remains unknown even whether or not there is any molecular biological feature that is altered in common in diverse cancers.

In cancers, there is no consistency in the combinations of altered cancer genes (Non-patent Documents 7 to 9), and it has been reported that alterations in signaling pathways rather than individual genes would facilitate tumorigenesis (Non-patent Document 10). Moreover, even in the case of common signaling pathways that are altered equally in cancers, different components are altered in different tumor tissues (Non-patent Documents 2 to 4).

Against this backdrop, enhanced expression of proteins, which are highly expressed in common in many cancer tissues, has been regarded merely as a minor change reflecting the phenotype characteristic of tumors (i.e., enhanced metabolic processes such as glycolysis, macromolecular synthesis and DNA replication) and intracellular stresses arising therefrom (Non-patent Documents 10 to 12). Thus, the enhanced expression of these proteins has received little attention as a target for cancer treatment or prevention.

Since conventional reagents for tumor detection are designed to use a marker specific for a particular type of tumor as an indicator for tumor detection, many kinds of test reagents should be used at the same time in order to comprehensively detect tumors which may develop in the patient's body. The use of many kinds of test reagents not only leads to a significant reduction in the cost-effectiveness ratio because of requiring high costs, but also raises a problem of an increased work burden on medical practitioners and an increased mental and physical burden on patients.

Moreover, most of the conventional reagents for tumor detection are designed to use a tumor marker appearing after tumor-induced phenotypic changes as an indicator for tumor detection, and hence have no ability to detect tumors at an early stage of tumorigenesis or at the precancerous stage. Thus, when tumors were detected with conventional detection reagents, the tumor diseases have already reached advanced stages, which disadvantageously limits the range of effective therapies which can be selected for treatment.
Non-patent Document 1: Tomatis, L., and Huff, J. (2001) Environ. Health Perspect. 109, A458-460
Non-patent Document 2: Parsons, D.W. et al. (2008) Science 321, 1807-1812
Non-patent Document 3: The Cancer Genome Atlas Research Network, 2008
Non-patent Document 4: Ding, L. et al. (2008) Nature 455, 1069-1075
Non-patent Document 5: Pleasance, E.D., et al. (2010) Nature 463, 184-190
Non-patent Document 6: Stephens, P.J. et al. (2009) Nature 462, 1005-1010
Non-patent Document 7: Stratton, M.R., Campbell, P.J., and Futreal, P.A. (2009) Nature 458, 719-724
Non-patent Document 8: Hahn, W.C., and Weinberg, R.A. (2002) Nat. Rev. Cancer 2, 331-341
Non-patent Document 9: Weber, B.L. (2002) Cancer Cell 1, 37-47
Non-patent Document 10:Vogelstein, B., and Kinzler, K.W. (2004) Nat. Med. 10, 789-799
Non-patent Document 11:Futreal, P.A., Coin, L. et al. (2004) Nat. Rev. Cancer 4, 177-183
Non-patent Document 12:Vander Heiden, M.G., Cantley, L.C., and Thompson, C.B. (2009) Science 324, 1029-1033

### DISCLOSURE OF THE INVENTION

Under these circumstances, there has been a demand for the development of a test reagent and a therapeutic pharmaceutical composition, each targeting a marker which is expressed equally in various types of tumors. Thus, the present invention aims to provide a test reagent and a therapeutic pharmaceutical composition, each targeting a marker which is expressed equally in various types of tumors or cancers. Moreover, the present invention also aims to provide a detection reagent allowing tumor detection at an early stage of tumorigenesis.

As a result of extensive and intensive efforts, the inventors of the present invention have found that the FEAT protein (faint expression in normal tissues, aberrant overexpression in tumors) is overexpressed in common from an early stage in diverse human cancer tissues. The inventors of the present invention have conducted expression microarray analyses and have found that FEAT induces oncogenic signaling pathways. Moreover, the inventors of the present invention have prepared transgenic (Tg) mice for overexpression of FEAT and have found that FEAT also promotes tumorigenesis *in vivo* in the experiments using these Tg mice. These findings led to the completion of the present invention.

The present invention therefore provides the following.
[1] A reagent for tumor detection, which comprises a probe for the FEAT gene or amplification primers for the FEAT gene, or an antibody against the FEAT protein or a fragment of the antibody.
[2] A pharmaceutical composition for tumor prevention, which comprises a substance shown in (a) or (c) below or cells shown in (b) below:
   (a) a substance capable of inhibiting FEAT gene expression or FEAT protein activity;
   (b) immune cells capable of reacting with the FEAT protein; or
   (c) a substance capable of binding to the FEAT protein, which substance carries an antitumor agent, the substance shown in (a) above or the cells shown in (b) above.
[3] The pharmaceutical composition according to [2] above, wherein the substance capable of inhibiting FEAT gene expression is siRNA against the FEAT gene.
[4] The pharmaceutical composition according to [2] above, wherein the substance capable of inhibiting FEAT protein activity is an antibody against the FEAT protein.
[5] The pharmaceutical composition according to [2] above, wherein the immune cells are T cells, B cells or dendritic cells.
[6] A method for tumor detection, which comprises reacting a test sample taken from a living body with the reagent according to [1] above.
[7] The method according to [6] above, wherein the test sample is blood, a body fluid or a tissue section.
[8] A tumor animal model, which consists of a transgenic non-human animal transformed with the FEAT gene.

The reagent of the present invention enables the detection of various types of cancers or precancerous lesions. Moreover, the reagent of the present invention also allows tumor detection at an early stage of tumorigenesis. Further, the pharmaceutical composition of the present invention allows effective treatment or prevention of tumors.

### BRIEF DESCRIPTION OF THE DRAWINGS

Figure 1 shows the results studied for process of biochemical purification, conservation across species and enzyme activity of the FEAT protein.
Figure 2 shows identification of caspase cleavage sites in the FEAT protein during apoptotic cell death, along with the structure of the human FEAT protein. In Figure 2F, the yellow highlighted regions in human FEAT each represent an S-adenosylmethionine (SAM)-binding motif.
Figure 3 shows inverse correlation between FEAT protein expression level and apoptosis, along with intracellular distribution of the FEAT protein.
Figure 4 shows the results of Western blotting indicating the expression of the FEAT protein in all the tested cell lines of human origin, along with the apoptosis-suppressing effect of wild-type FEAT, mutant FEAT and FEAT fragments.
Figure 5 shows the results of Western blotting indicating FEAT expression in human normal and tumor tissues, along with FEAT protein-mediated induction of oncogenic signaling pathways.
Figure 6 shows the results of Western blotting indicating FEAT expression in various organs of non-Tg and Tg mice, decreased apoptotic cell death in thymocytes of Tg mice, and the frequency of tumor development in Tg mice.
Figure 7 shows the gross appearance of tumors developed in Tg mice (indicated with arrows), along with the tumor-free survival of these mice.
Figure 8 shows the tumor-free survival of Tg mice and non-Tg littermates (A), histological features in primary liver cancer focus (B) and in lung metastatic lesions (C), as well as the human chromosomal distribution of genes with copy number alterations detected by array-CGH on liver cancer in FEAT Tg mice (D).
Figure 9 shows photomicrographs of various tumor tissues developed in FEAT Tg mice.
Figure 10A shows liver cancer-induced amplifications (red) and deletions (green) in mouse chromosomes, as detected by array-CGH.
Figure 10B is continued from Figure 10A.
Figure 11 shows the immunohistochemical observation of FEAT expression in various human normal and cancer tissues.
Figure 12 shows enhanced FEAT expression in various human cancer tissues.
Figure 13 shows enhanced FEAT expression in liver cirrhosis as precancerous lesions adjacent to liver cancer.

### BEST MODES FOR CARRYING OUT THE INVENTION

The present invention will be described in more detail below. The following embodiments are illustrated to describe the present invention, and it is not intended to limit the present invention only to these embodiments. The present invention can be implemented in various modes, without departing from the spirit of the present invention.

It should be noted that all publications cited herein, including prior art documents, patent gazettes and other patent documents, are incorporated herein by reference. Moreover, this specification incorporates the contents disclosed in the specification and drawings of Japanese Patent Application No. 2010-181835 (filed on August 16, 2010), based on which the present application claims priority.

The inventors of the present invention have found that the FEAT protein is overexpressed in diverse tumor tissues and contributes to tumorigenesis.

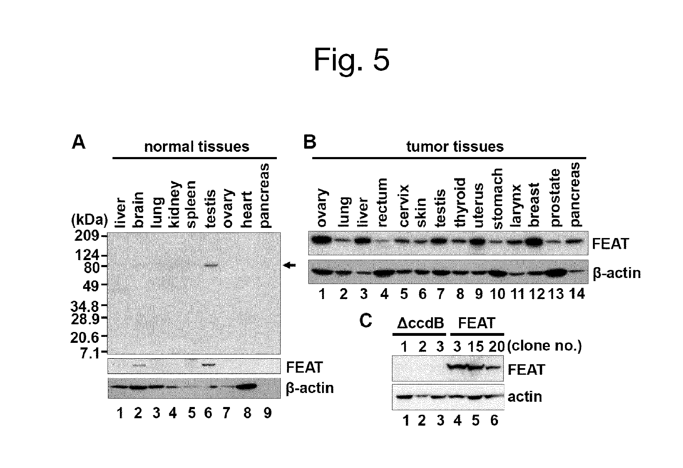

In the case of normal tissues, the FEAT gene and protein are not expressed in almost all tissues and are weakly expressed only in the testis, brain and liver. On the other hand, the FEAT gene is expressed equally in diverse tumor tissues including ovarian cancer, lung cancer, liver cancer, rectal cancer, uterine cervical cancer, skin cancer, testicular cancer, thyroid cancer, uterine cancer, gastric cancer, pharyngeal cancer, breast cancer, prostate cancer, and pancreatic cancer (Figure 5B).

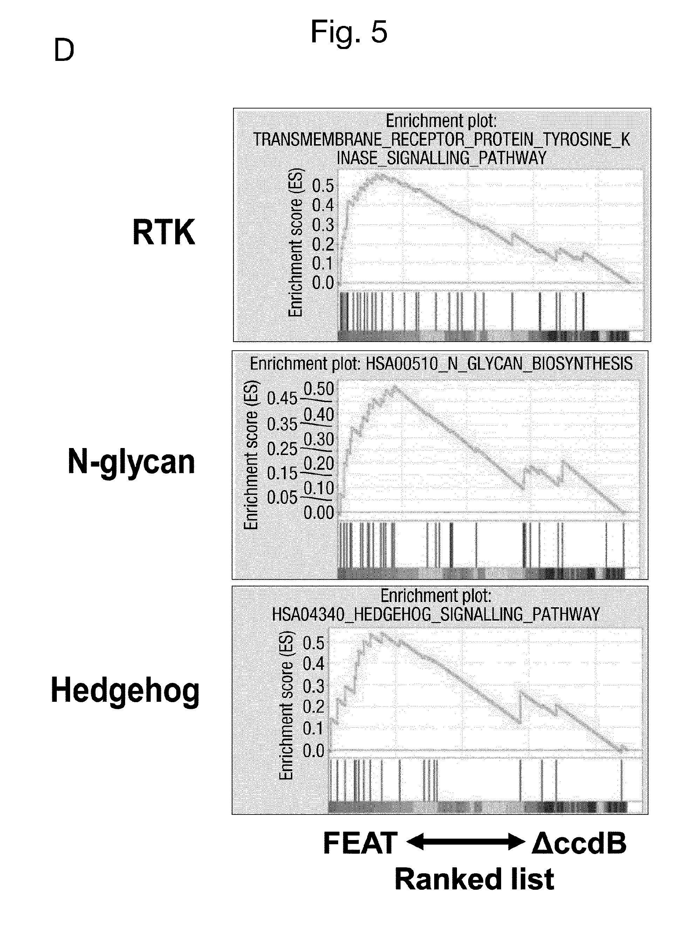

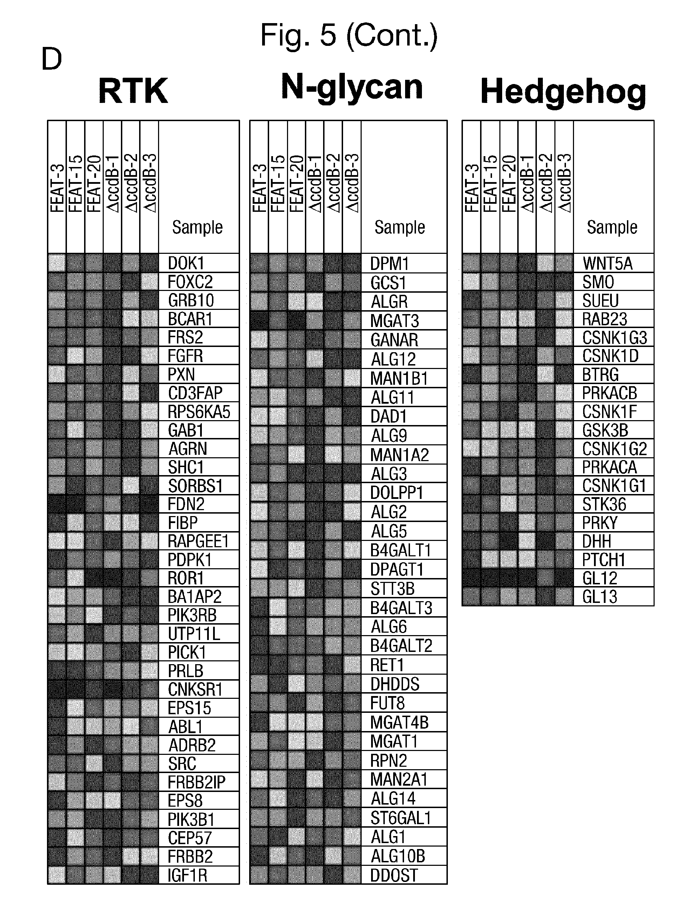

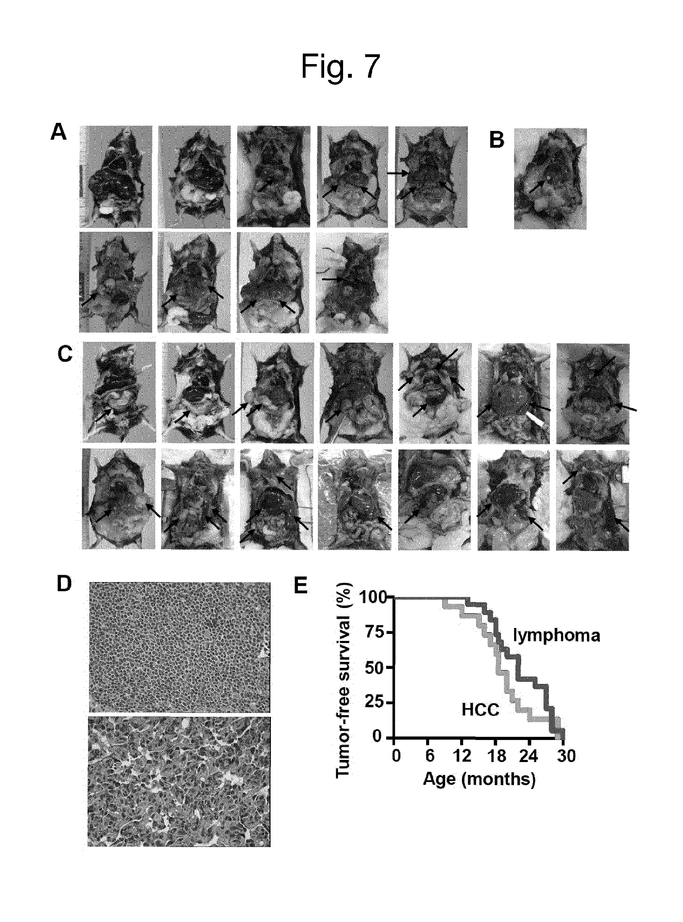

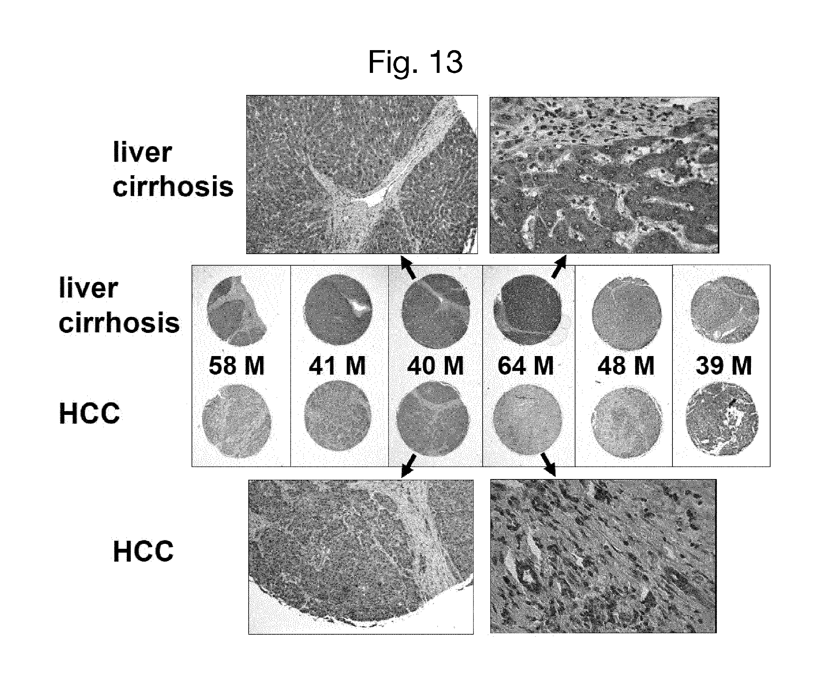

The inventors of the present invention have found that the FEAT protein is produced prior to tumor-induced phenotypic changes during carcinogenesis in humans (Figure 13). Moreover, the inventors of the present invention have also found that the FEAT protein, when overexpressed in cultured cells, activates the receptor tyrosine kinase signaling pathway and the Hedgehog signaling pathway among oncogenic pathways (Figure 5D), and have further confirmed that tumor tissues are formed in various organs of transgenic mice engineered to overexpress the FEAT protein (Figures 7A to 7C).

Thus, even in the case of cells which have not yet turned into tumors, those expressing the FEAT protein are highly likely to turn into tumors in the future, i.e., can be regarded as precancerous cells.

An explanation will now be made of FEAT, which is used as a tumor marker in the present invention.

The FEAT protein is a protein encoded by a gene called "comparative gene identification-01 (CGI-01)," "KIAA0859" or "methyltransferase like 13 (METTL13)."

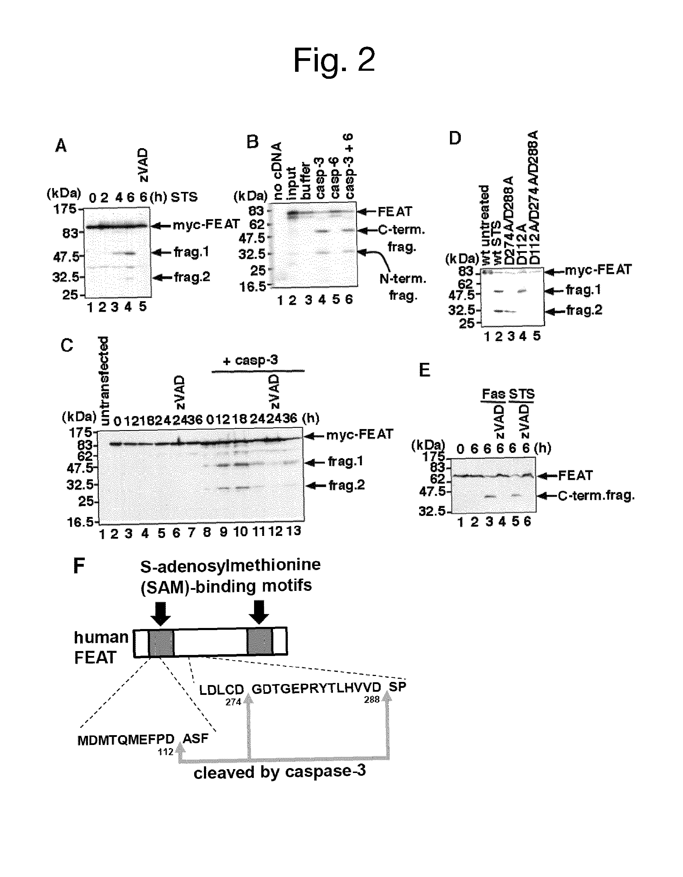

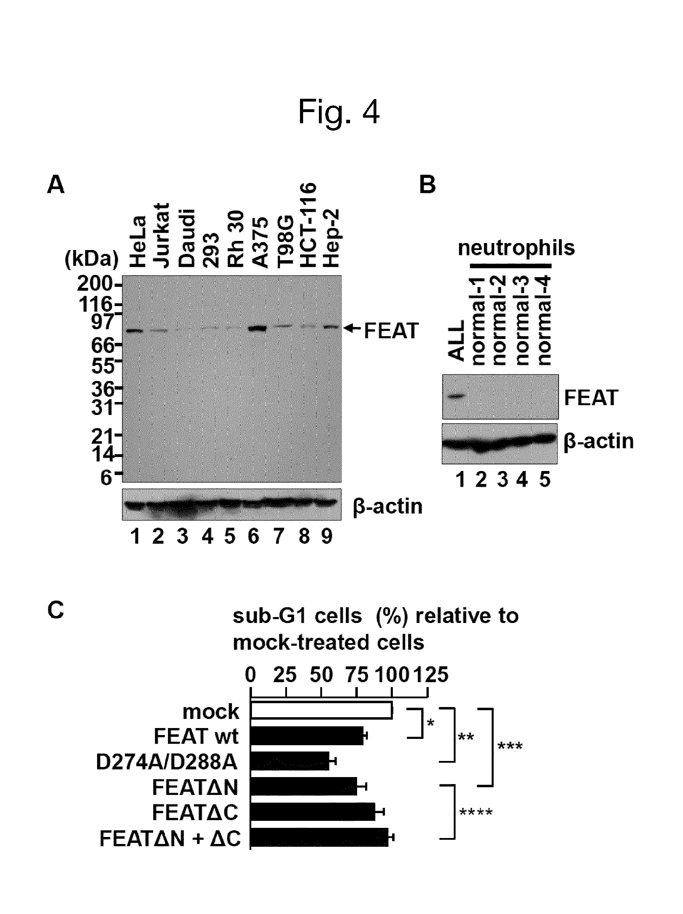

The FEAT protein is cleaved by caspase-3, but not by caspase-6 (Figure 2B). In addition, the FEAT protein has the function of suppressing apoptosis in cells (Figure 4C).

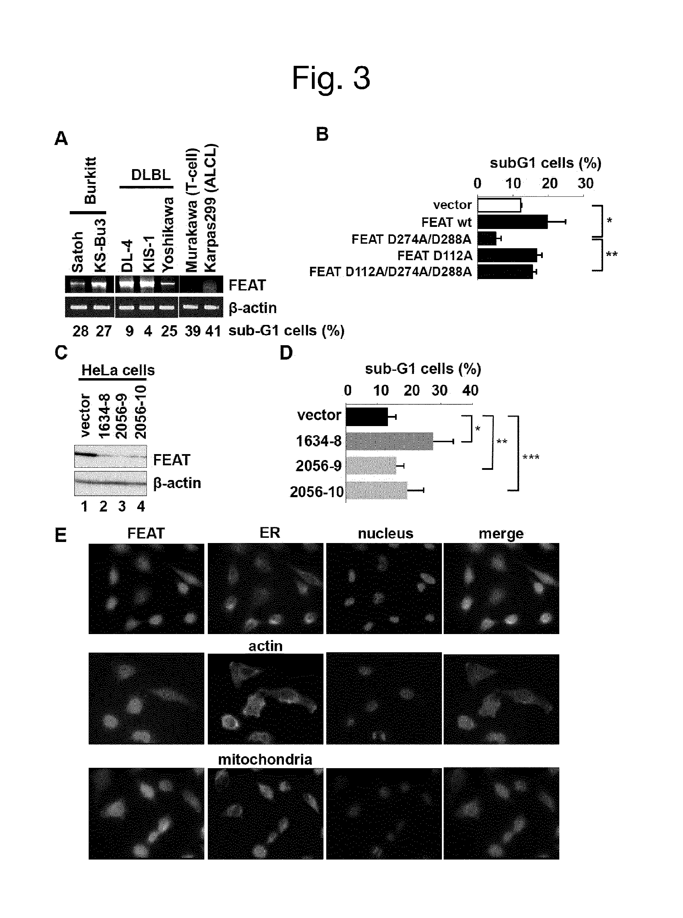

The nucleotide sequence of the human FEAT gene and the amino acid sequence of the human FEAT protein are as shown in SEQ ID NO: 1 (Accession Number: NM_015935) and SEQ ID NO: 2, respectively. Moreover, not only the wild-type FEAT protein (SEQ ID NO: 2), but also mutants thereof, such as those carrying either or both a D274A mutation in which alanine substitutes for aspartic acid at position 274 (SEQ ID NO: 2) in the caspase-3 cleavage sites and a D288A mutation in which alanine substitutes for aspartic acid at position 288 (SEQ ID NO: 2) in the caspase-3 cleavage sites, are resistant to caspase-3 cleavage and hence have a stronger apoptosis-suppressing function (Figure 3B). Furthermore, not only the full-length FEAT protein (SEQ ID NO: 2), but also fragments thereof, particularly fragments comprising a sequence composed of amino acids at positions 289 to 699 in the amino acid sequence of the FEAT protein, have an apoptosis-suppressing function.

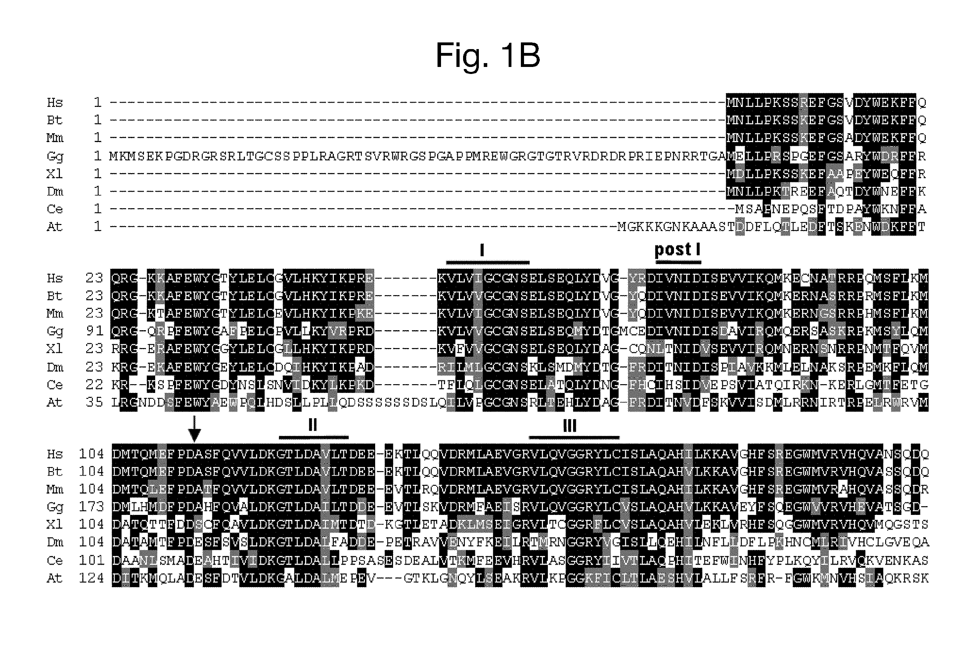

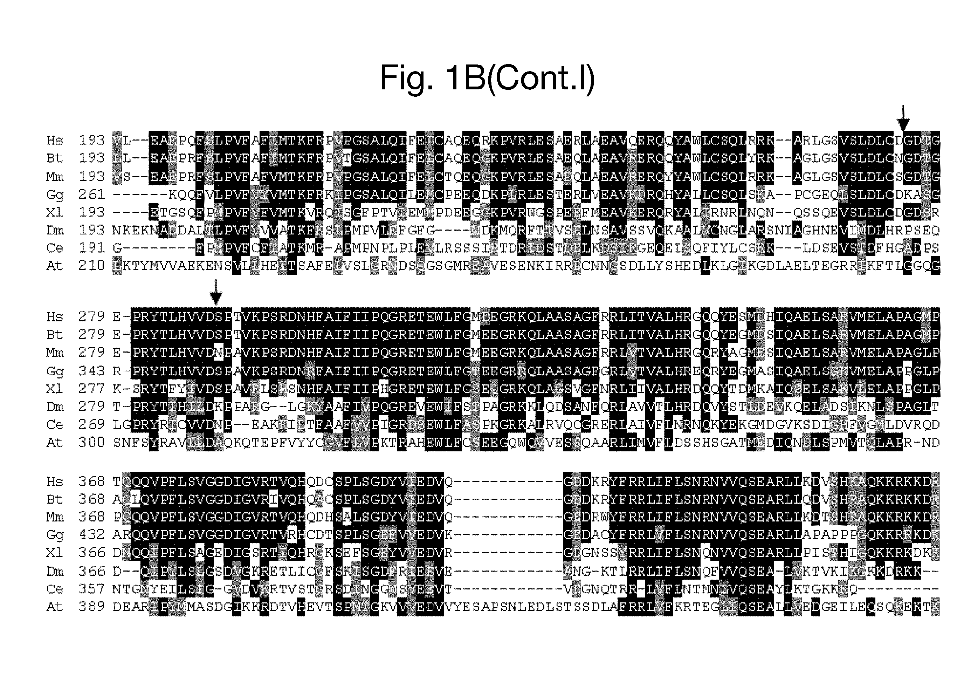

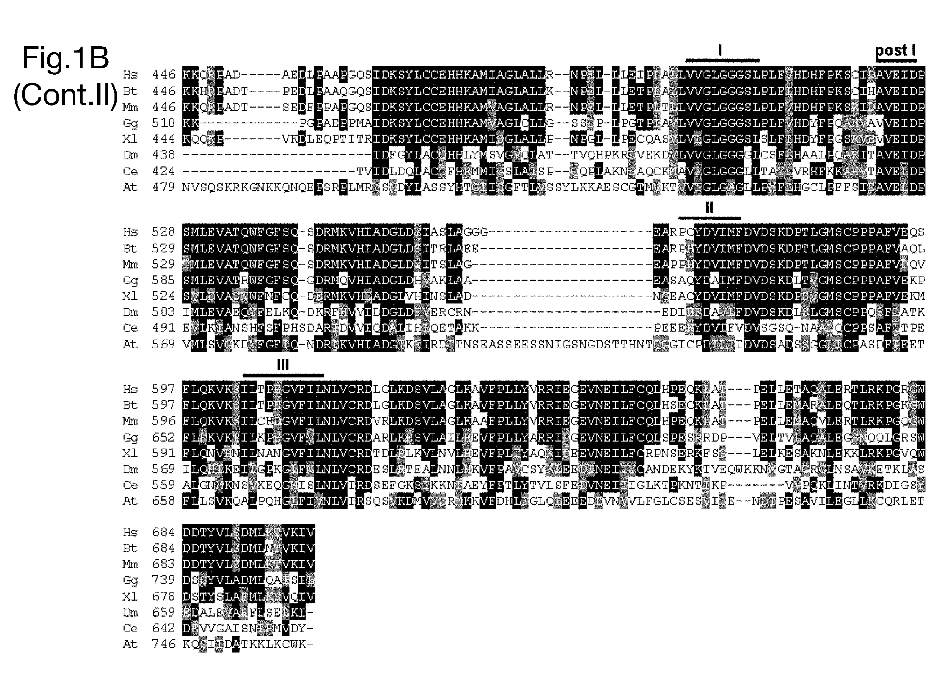

In addition, the FEAT protein contains two S-adenosylmethionine-binding motifs (SAM-binding motifs). The SAM-binding motif is a motif characteristic of methyltransferase and related enzymes thereof (Figure 1B). However, it has remained unknown whether or not the FEAT protein has methyltransferase activity. As consensus sequences in SAM-binding motifs, the following sequences can be listed, by way of example.

| | Methyltransferase Sequence Motifs I-III | |
|---|---|---|
| Motif I | Motif II | Motif III |
| | | V |
| L | A Y L | L I |
| IVEV C P | GTY VIV | K IIFL |
| VLDIGGGTG | PQFDAIFC | LLRPGGRLLI |

The above FEAT protein and FEAT gene can be obtained by the procedures described later in the Example section, known genetic engineering procedures, known synthesis procedures, etc.

### 1. Reagent for tumor detection

The present invention provides a reagent for tumor detection, which comprises a probe for the FEAT gene or amplification primers for the FEAT gene, or an antibody against the FEAT protein or a fragment of the antibody.

The FEAT gene or the FEAT protein are expressed widely and specifically in tumor cells or precancerous cells, but are not expressed in most normal cells. Thus, if the FEAT gene or the FEAT protein is detected in test cells, such test cells can be diagnosed as tumor cells or precancerous cells.

As used herein, the term "FEAT gene" is intended to include the above human wild-type FEAT gene having the nucleotide sequence shown in SEQ ID NO: 1, as well as FEAT homolog genes derived from non-human species and mutant genes thereof.

More specifically, examples of the FEAT gene include any one polynucleotide selected from the group consisting of (a) to (e) shown below:
(a) a polynucleotide comprising the nucleotide sequence shown in SEQ ID NO: 1;
(b) a polynucleotide encoding a protein which consists of the amino acid sequence shown in SEQ ID NO: 2;
(c) a polynucleotide encoding a protein which comprises an amino acid sequence with deletion, substitution or addition of one or several amino acids in the amino acid sequence shown in SEQ ID NO: 2 and which has a tumor-promoting function;
(d) a polynucleotide encoding a protein which has an amino acid sequence sharing an identity of 60% or more with the amino acid sequence of SEQ ID NO: 2 and which has a tumor-promoting function; and
(e) a polynucleotide which is hybridizable under stringent conditions with a polynucleotide consisting of a nucleotide sequence complementary to the nucleotide sequence shown in SEQ ID NO: 1 and which encodes a protein having a tumor-promoting function.

Among the above polynucleotides (a) to (e), (a) and (b) each correspond to the human wild-type FEAT gene, while (c) to (e) correspond to FEAT homolog genes derived from non-human species and mutant FEAT genes. The FEAT gene may be either DNA or RNA (messenger RNA).

As used herein, the expression "polynucleotide which is hybridizable under stringent conditions" is intended to mean, for example, a polynucleotide that can be obtained by means of colony hybridization, plaque hybridization, Southern hybridization or other hybridization techniques using, as a probe, the whole or a part of a polynucleotide consisting of a nucleotide sequence complementary to the nucleotide sequence shown in SEQ ID NO: 1 or a polynucleotide consisting of a nucleotide sequence encoding the amino acid sequence shown in SEQ ID NO: 2. For hybridization, it is possible to use techniques as described in, e.g., "Sambrook & Russell, Molecular Cloning: A Laboratory Manual Vol. 3, Cold Spring Harbor, Laboratory Press 2001" and "Ausubel, Current Protocols in Molecular Biology, John Wiley & Sons 1987-1997."

As used herein, the term "stringent conditions" may be any of low stringent conditions, moderately stringent conditions and high stringent conditions. "Low stringent conditions" refer to, for example, conditions of 5 × SSC, 5 × Denhardt's solution, 0.5% SDS, 50% formamide and 32°C. Likewise, "moderately stringent conditions" refer to, for example, conditions of 5 × SSC, 5 × Denhardt's solution, 0.5% SDS, 50% formamide and 42°C. "High stringent conditions" refer to, for example, conditions of 5 × SSC, 5 × Denhardt's solution, 0.5% SDS, 50% formamide and 50°C. Under these conditions, it can be expected that DNA having a higher identity is efficiently obtained at a higher temperature. However, the stringency of hybridization would be affected by a plurality of factors, including temperature, probe concentration, probe length, ionic strength, reaction time, salt concentration and so on. Those skilled in the art would be able to achieve the same stringency by selecting these factors as appropriate.

It should be noted that if a commercially available kit is used for hybridization, an Alkphos Direct Labelling and Detection System (GE Healthcare) may be used for this purpose, by way of example. In this case, hybridization may be accomplished in accordance with the protocol attached to the kit, i.e., a membrane may be incubated overnight with a labeled probe and then washed with a primary washing buffer containing 0.1% (w/v) SDS under conditions of 55°C to detect the hybridized DNA.

In addition to those listed above, other hybridizable polynucleotides include polynucleotides sharing an identity of 50% or more, 60% or more, 70% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more with a polynucleotide consisting of the nucleotide sequence shown in SEQ ID NO: 1 or with a polynucleotide consisting of a nucleotide sequence encoding the amino acid sequence shown in SEQ ID NO: 2, as calculated by homology search software such as FASTA or BLAST using default parameters.

The identity of nucleotide sequences can be determined by using FASTA (Science 227 (4693): 1435-1441, (1985)) or the algorithm of Karlin and Altschul, BLAST (Basic Local Alignment Search Tool) (Proc. Natl. Acad. Sci. USA 872264-2268, 1990; Proc Natl Acad Sci USA 90: 5873, 1993). Based on the algorithm of BLAST, programs called BLASTN, BLASTX and BLASTP have been developed (Altschul SF, et al: J Mol Biol 215: 403, 1990). If BLASTN is used for nucleotide sequence analysis, parameters may be set to, for example, score = 100 and wordlength = 12.

The polynucleotides of the present invention described above can be obtained by known genetic engineering procedures or known synthesis procedures.

In the present invention, examples of a probe for the FEAT gene (hereinafter referred to as "the probe of the present invention") or amplification primers for the FEAT gene (hereinafter referred to as "the primers of the present invention") include oligonucleotides which are hybridizable under stringent conditions with the FEAT gene. "Stringent conditions" are as described above. The term "oligonucleotide" is intended to mean a nucleotide chain having a length of preferably 5 to 500 bp, more preferably 10 to 200 bp, and even more preferably 10 to 100 bp. These oligonucleotides may be readily synthesized by using various automatic synthesizers (e.g., AKTA oligopilot plus 10/100 (GE Healthcare)), or alternatively, their synthesis may be entrusted to a third party (e.g., Promega or Takara), etc.

When the reagent of the present invention comprises a probe for the FEAT gene, assay methods for tumor detection include Northern blotting (Sambrook, Fritsch and Maniatis, "Molecular Cloning: A Laboratory Manual" 2nd Edition (1989), Cold Spring Harbor Laboratory Press), microarrays (Affymetrix; see United States Patent Nos. 6,045,996, 5,925,525 and 5,858,659) or TaqMan PCR (Sambrook, Fritsch and Maniatis, "Molecular Cloning: A Laboratory Manual" 2nd Edition (1989), Cold Spring Harbor Laboratory Press) for the FEAT gene expressed in the tumor. Likewise, when the reagent of the present invention comprises primers for the FEAT gene, methods for tumor detection include PCR amplification or sequencing (Sambrook, Fritsch and Maniatis, "Molecular Cloning: A Laboratory Manual" 2nd Edition (1989), Cold Spring Harbor Laboratory Press) for the FEAT gene expressed in the tumor. The reagent of the present invention may comprise both the probe and the primers.

In the context of the present invention, the term "FEAT protein" is intended to include not only the human wild-type FEAT protein consisting of the amino acid sequence shown in SEQ ID NO: 2, but also FEAT homolog proteins derived from non-human species and mutant proteins thereof. In addition, among fragments generated by cleavage of the FEAT protein, those having a tumor-promoting function also fall within the FEAT protein.

Specific examples of the FEAT protein include polypeptides shown in (f), (g) and (h) below or fragments thereof:
(f) a polypeptide which comprises the amino acid sequence shown in SEQ ID NO: 2;
(g) a polypeptide which comprises an amino acid sequence with deletion, substitution or addition of one or several amino acids in the amino acid sequence shown in SEQ ID NO: 2 and which has a tumor-promoting function; and
(h) a polypeptide which comprises an amino acid sequence sharing an identity of 60% or more with the amino acid sequence shown in SEQ ID NO: 2 and which has a tumor-promoting function.

The above polypeptide (g) or (h) is typically a FEAT homolog protein derived from non-human species or a mutant protein of the wild-type FEAT protein, but it also includes other polypeptides based on a polypeptide consisting of the amino acid sequence shown in SEQ ID NO: 2, which may be artificially obtained by site-directed mutagenesis as described in "Sambrook & Russell, Molecular Cloning: A Laboratory Manual Vol. 3, Cold Spring Harbor Laboratory Press 2001," "Ausubel, Current Protocols in Molecular Biology, John Wiley & Sons 1987-1997," "Nuc. Acids. Res., 10, 6487(1982)," "Proc. Natl. Acad. Sci. USA, 79, 6409(1982)," "Gene, 34, 315 (1985)," "Nuc. Acids. Res., 13, 4431(1985)," "Proc. Natl. Acad. Sci. USA, 82, 488(1985)," etc.

As used herein, the expression "polypeptide which comprises an amino acid sequence with deletion, substitution or addition of one or several amino acids in the amino acid sequence shown in SEQ ID NO: 2 and which has a tumor-promoting function" is intended to include polypeptides which comprise an amino acid sequence with deletion, substitution or addition of, e.g., 1 to 100 amino acid residues, 1 to 90 amino acid residues, 1 to 80 amino acid residues, 1 to 70 amino acid residues, 1 to 60 amino acid residues, 1 to 50 amino acid residues, 1 to 40 amino acid residues, 1 to 30 amino acid residues, 1 to 20 amino acid residues, 1 to 10 amino acid residues, 1 to 9 amino acid residues (one or several amino acid residues), 1 to 8 amino acid residues, 1 to 7 amino acid residues, 1 to 6 amino acid residues, 1 to 5 amino acid residues, 1 to 4 amino acid residues, 1 to 3 amino acid residues, 1 or 2 amino acid residues, or a single amino acid residue in the amino acid sequence shown in SEQ ID NO: 2 and which have a tumor-promoting function. In general, a smaller number is more preferred for the above deletion, substitution or addition of amino acid residues.

Moreover, examples of such polypeptides include those which have an amino acid sequence sharing an identity of 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, 99% or more, 99.1% or more, 99.5% or more, or 99.9% or more with the amino acid sequence shown in SEQ ID NO: 2 and which have a tumor-promoting function. In general, a larger value is more preferred for the above identity.

The identity of amino acid sequences can be determined by using FASTA (Science 227 (4693): 1435-1441, (1985)) or the algorithm of Karlin and Altschul, BLAST (Basic Local Alignment Search Tool) (Proc. Natl. Acad. Sci. USA 872264-2268, 1990; Proc Natl Acad Sci USA 90: 5873, 1993). Based on the algorithm of BLAST, programs called BLASTN, BLASTX and BLASTP have been developed (Altschul SF, et al: J Mol Biol 215: 403, 1990). BLASTP is generally used for amino acid sequence analysis, and parameters may be set to, for example, score = 60 and wordlength = 3 if amino acids having an identity of 60% or more are searched during analysis of amino acid sequence identity.

Whether or not a test protein has a tumor-promoting function can be confirmed by introducing the test protein or a gene thereof into appropriate normal host cells (e.g., human neutrophils) which do not express FEAT, incubating the host cells under appropriate culture conditions, and then observing tumorigenesis in the host cells.

In a certain embodiment, the tumor-promoting function may be an apoptosis-suppressing function. Whether or not a test protein has an apoptosis-suppressing function can be confirmed by introducing the test protein or a gene thereof into appropriate host cells (e.g., human neutrophils) which do not express FEAT, incubating the host cells under appropriate culture conditions, and then analyzing the amount of apoptotic cells with fragmented DNA among the host cells by flow cytometry to determine apoptotic cell counts.

In the present invention, in embodiments where one or more amino acid residues are deleted, substituted or added, deletion, substitution or addition of one or several amino acid residues may occur at any one or more positions in the same sequence, or alternatively, two or more of deletion, substitution and addition may occur at the same time. Moreover, in a case where addition of an amino acid residue(s) occurs, the amino acid residue(s) may be added by being inserted into the amino acid sequence or may be added to the N-terminal or C-terminal end of the amino acid sequence.

Examples of interchangeable amino acid residues are shown below. Amino acid residues included in the same group are interchangeable with each other.
Group A: leucine, isoleucine, norleucine, valine, norvaline, alanine, 2-aminobutanoic acid, methionine, o-methylserine, t-butylglycine, t-butylalanine, cyclohexylalanine;
Group B: aspartic acid, glutamic acid, isoaspartic acid, isoglutamic acid, 2-aminoadipic acid, 2-aminosuberic acid;
Group C: asparagine, glutamine;
Group D: lysine, arginine, ornithine, 2,4-diaminobutanoic acid, 2,3-diaminopropionic acid;
Group E: proline, 3-hydroxyproline, 4-hydroxyproline;
Group F: serine, threonine, homoserine;
Group G: phenylalanine, tyrosine.

The above FEAT proteins may also be prepared by chemical synthesis methods such as Fmoc method (fluorenylmethyloxycarbonyl method) and tBoc method (t-butyloxycarbonyl method). Alternatively, they may be chemically synthesized by using peptide synthesizers commercially available from Advanced Automation Peptide Protein Technologies, Perkin Elmer, Protein Technologies, PerSeptive, Applied Biosystems, SHIMADZU, etc.

Although the FEAT protein is expressed in tumor cells or precancerous cells, fragments of the FEAT protein may also be contained in tumor cells or precancerous cells because the FEAT protein will be cleaved by intracellular caspase-3 once apoptotic cell death has occurred. Thus, the reagent of the present invention is configured to detect the full-length FEAT protein or a fragment thereof.

Such a fragment is not limited in any way, but is preferably a fragment comprising a sequence consisting of amino acids at positions 289 to 699 in the amino acid sequence shown in SEQ ID NO: 2. This fragment is preferred in terms of having a tumor-promoting function, albeit weaker than that of the full-length protein.

An antibody against the FEAT protein (or a fragment thereof) (hereinafter referred to as "the antibody of the present invention") may be either polyclonal or monoclonal.

### (1) Preparation of polyclonal antibodies

To prepare the above antibody, the FEAT protein is first prepared for use as an immunogen (antigen). The FEAT protein serving as an antigen may be prepared in a genetic engineering manner on the basis of the amino acid sequence of the FEAT protein.

The resulting purified FEAT is then used to immunize animals. Immunization may be accomplished by administering a solution containing the FEAT protein to mammals (e.g., mice, rats, rabbits, guinea pigs, goats or the like). Administration may be accomplished primarily by intravenous, subcutaneous or intraperitoneal injection. If necessary, an adjuvant may also be added as appropriate. Subsequently, sera (antisera) obtained as a result of immunization are collected. Serum collection is not limited in any way and sera may be collected in a routine manner form the blood of the immunized animals at 1 to 28 days after the final administration. The collected antisera are screened by known immunoassay techniques to select a desired antiserum. Antibodies contained in the desired antiserum obtained by the above screening are polyclonal antibodies. If these antibodies are required to be purified, known purification techniques such as salting out with ammonium sulfate, ion exchange chromatography, affinity chromatography, gel chromatography and so on may be used either alone or in combination for this purpose.

### (2) Preparation of monoclonal antibodies

Preparation of an antigen and a solution thereof, as well as immunization therewith may be accomplished in the same manner as described above for preparation of polyclonal antibodies.

At 1 to 14 days after the final administration of the antigen, antibody-producing cells are collected. As antibody-producing cells, preferred are spleen cells, lymph node cells, peripheral blood cells and the like, by way of example, and more preferred are spleen cells. Cell fusion is induced between the collected antibody-producing cells and myeloma cells to thereby obtain fused cells (hybridomas).

Myeloma cells used for this purpose include, for example, PAI, P3X63-Ag.8.U1(P3U1), NS-I and so on. The above cell fusion may be accomplished, for example, by mixing the antibody-producing cells with the myeloma cells in a medium for animal cell culture (e.g., RPMI-1640 medium) to cause fusion reaction. In general, fusion reaction is preferably performed in the presence of a cell fusion promoter such as polyethylene glycol. Alternatively, a commercially available electroporation-based cell fusion apparatus may also be used to fuse the antibody-producing cells with the myeloma cells.

The cells obtained after fusion reaction are cultured, e.g., in HAT selective medium. After being cultured as above, cells grown in the HAT selective medium are fused cells (hybridomas). The hybridomas obtained by being cultured as above are screened to select desired hybridomas, followed by known purification techniques such as salting out with ammonium sulfate, ion exchange chromatography, affinity chromatography, gel chromatography and so on, which may be used either alone or in combination as appropriate, to thereby purify monoclonal antibodies from the above hybridomas. As to the preparation of monoclonal antibodies, reference may be made to United States Patent No. 4,816,567.

Moreover, in the present invention, a humanized antibody, a human antibody, or a fragment thereof may also be used.

A humanized antibody refers to a monoclonal antibody prepared in a genetic engineering manner, and more specifically refers to an antibody designed such that the complementarity determining regions (CDRs) in its hypervariable regions are composed partially or completely of complementarity determining regions derived from mouse monoclonal antibody hypervariable regions, the framework regions (FRs) in its variable regions are composed of FRs derived from human immunoglobulin variable regions, and its constant regions are composed of human immunoglobulin constant regions.

A human antibody refers to an antibody whose immunoglobulin-constituting regions are all derived from genes encoding human immunoglobulin sequences.

As to the preparation of humanized and human antibodies, reference may be made to the following documents: Riechmann L. et al. (1988) Nature 332 (6162): 332-323; and Japanese Patent No. 2912618.

### (3) Antibody fragments

An antibody fragment is intended to mean a partial fragment comprising an antigen-binding region of the above antibody, and specific examples include F(ab')₂, Fab', Fab, Fv (variable fragment of antibody), scFv (single chain Fv), dsFv (disulphide stabilized Fv) and so on.

When the reagent of the present invention comprises an antibody against the FEAT protein, assay methods for tumor detection include immunohistological staining (Lorette C. Javois ed. "Immunocytochemical Methods and Protocols (Methods in Molecular Biology)" Humana Press; 2nd edition (February 15, 1999)), immunoprecipitation, affinity chromatography, pull-down assay, enzyme linked immunosorbent assay (ELISA) and Western blotting (Gary C. Howard and Matthew R. Kaser ed. "Making and Using Antibodies: A Practical Handbook" CRC Press; 1st edition (December 13, 2006)), antibody microarrays (Chaga GS (2008). "Antibody arrays for determination of relative protein abundances." Methods Mol. Biol. 441: 129-51; Rivas LA, García-Villadangos M, Moreno-Paz M, Cruz-Gil P, Gómez-Elvira J, Parro V (November 2008). "A 200-antibody microarray biochip for environmental monitoring: searching for universal microbial biomarkers through immunoprofiling." Anal. Chem. 80 (21): 7970-9) for the FEAT protein expressed in the tumor.

### 2. Pharmaceutical composition for tumor prevention

The inventors of the present invention have found that the FEAT protein is expressed from a very early stage (prior to tumor-induced phenotypic changes) during the tumorigenesis process, and have further found that the expression of the FEAT protein promotes tumorigenesis.

Thus, when destroying not only FEAT-expressing cells which have already turned into tumors, but also FEAT-expressing cells which are in the precancerous stage, it is possible to inhibit tumor development and to prevent tumorigenesis.

Moreover, the inventors of the present invention have also found that the FEAT protein per se has an apoptosis-suppressing function and that the expression of the FEAT protein activates oncogenic signaling pathways.

Thus, tumorigenesis can also be prevented when functions of the FEAT protein or a gene thereof are inhibited to induce apoptotic cell death in tumor cells or precancerous cells.

Accordingly, the present invention provides a pharmaceutical composition for tumor prevention, which comprises a substance shown in (i) or (ii) below or cells shown in (iii) below:
(i) a substance capable of inhibiting FEAT gene expression or FEAT protein activity;
(ii) immune cells capable of reacting with the FEAT protein; or
(iii) a substance capable of binding to the FEAT protein, which substance carries an antitumor agent, the substance shown in (i) above or the cells shown in (ii) above.

### (i-1) Substance capable of inhibiting FEAT gene expression

Specific examples of the "substance capable of inhibiting FEAT gene expression" shown in (i) above include substances capable of reducing the expression level of messenger RNA (mRNA) for the FEAT gene, as exemplified by low molecular compounds, hormones, proteins and nucleic acids. In one embodiment, such a substance may be a nucleic acid capable of suppressing the functions or expression of the FEAT gene. Examples of such a nucleic acid include hairpin-shaped shRNAs (short hairpin RNAs) or double-stranded RNAs (dsRNAs) which produce siRNAs (small interfering RNAs) for RNA interference (RNAi), as well as antisense nucleic acids, decoy nucleic acids, or aptamers, etc. These inhibitory nucleic acids are able to suppress the expression of the above gene. The nucleotide sequence of the FEAT gene to be inhibited is as described above, and sequence information can be obtained for each case. In the present invention, the nucleotide sequence of the FEAT gene is as shown in SEQ ID NO: 1, although not only a coding region of FEAT, but also a non-coding region may be used as a target.

### (1) RNA interference

The inventors of the present invention have succeeded in knocking down FEAT expression in host cells by RNAi using shRNAs (Figure 3C) and thereby promoting apoptosis in the host cells (Figure 3D).

RNAi is a multi-step process proceeding through a number of stages. First of all, dsRNA or shRNA expressed from an RNAi expression vector is recognized by Dicer and cleaved into siRNAs of 21 to 23 nucleotides. These siRNAs are then integrated into an RNAi targeting complex, which is called the RNA-induced silencing complex (RISC), and the complexes between RISC and siRNAs bind to target mRNA containing sequences complementary to the siRNA sequences and thereby cleave the mRNA. The target mRNA is cleaved in the center of its region complementary to the siRNA, finally leading to rapid degradation of the target mRNA and reduced protein expression levels. The most potent siRNA duplexes are known to be sequences of 21 nucleotides in length, each comprising a 19 bp duplex with an overhang of two uridine residues at the 3'-terminal end (Elbashir S.M. et al., Genes and Dev, 15, 188-200 (2001)).

In general, a target sequence on mRNA may be selected from the cDNA sequence corresponding to the mRNA. However, the present invention is not limited to this region.

siRNA molecules may be designed on the basis of the criteria well known in the art. For example, as a target segment in target mRNA, it is possible to select a segment covering 15 to 30 contiguous bases, preferably 19 to 25 contiguous bases, preferably starting with AA, TA, GA or CA. siRNA molecules have a GC ratio of 30% to 70%, preferably 35% to 55%. Alternatively, a target sequence for RNAi may be selected as appropriate as described in Ui-Tei K. et al. ((2004) Nucleic Acids Res. 32, 936-948). For example, in the case of RNAi against the human FEAT gene, the following nucleotide sequences of 19 base pairs starting from positions 183, 259, 1385, 1550, 1634 and 2056 in the open reading frame region of FEAT cDNA (SEQ ID NOs: 3, 4, 5, 6, 7 and 8, respectively) can be used as targets for RNAi to thereby suppress FEAT gene expression.
5'-ACTGAGTGAGCAACTGTAT-3' (SEQ ID NO: 3)
5'-ATGAAGGAATGTAATGCCA-3' (SEQ ID NO: 4)
5'-AGTCCATTGATAAGAGTTA-3' (SEQ ID NO: 5)
5'-AGTCCTGCATTGATGCTGT-3' (SEQ ID NO: 6)
5'-TGAAGGTCCACATTGCAGA-3' (SEQ ID NO: 7)
5'-ACGTATGTCTTGTCAGATA-3' (SEQ ID NO: 8)

For introduction of siRNA into cells, it is possible to use, e.g., procedures in which synthesized siRNA is ligated to plasmid DNA and then introduced into cells, or procedures in which double-stranded RNA is annealed.

In the present invention, shRNA may also be used for providing RNAi effect. shRNA is an RNA molecule called short hairpin RNA, which has a stem-loop structure because some single-stranded regions form complementary strands with other regions.

shRNA may be designed to form a stem-loop structure as a part thereof. For example, assuming that a sequence covering a certain region is designated as sequence A, and a strand complementary to the sequence A is designated as sequence B, shRNA is design to comprise the sequence A, a spacer and the sequence B linked in this order on a single RNA strand and to have an overall length of 45 to 60 bases. The spacer may also have any length.

Although the sequence A is a sequence covering a partial region of the target FEAT gene, there is no particular limitation on the target region and any region may be selected as a candidate for the target region. In addition, the sequence A has a length of 19 to 25 bases, preferably 19 to 21 bases.

Further, in the present invention, microRNA may be used to inhibit FEAT gene expression. microRNA (miRNA) is an intracellular single-stranded RNA molecule having a length of about 20 to 25 bases and is a kind of ncRNA (non-coding RNA) which is considered to have the function of regulating the expression of other genes. miRNA is generated through processing upon transcription into RNA and is present as a nucleic acid capable of forming a hairpin structure which suppresses the expression of a target sequence.

Since miRNA is also an inhibitory nucleic acid based on RNAi, miRNA may also be designed and synthesized in the same manner as in the case of shRNA or siRNA.

Expression vectors for RNAi may be readily prepared with a commercially available DNA/RNA synthesizer (e.g., Applied Biosystems model 394) on the basis of pMuniH1 plasmid, pSINsi vector (Takara Bio Inc., Japan), pSIF1-H1 (System Biosciences, Inc.), etc. Alternatively, expression vectors for RNAi may be prepared by entrusting their preparation to third parties such as Cosmo Bio Co., Ltd. (Japan), Takara Bio Inc. (Japan), Invitrogen, Promega, etc.

### (2) Antisense nucleic acid

In another embodiment of the present invention, an antisense nucleic acid may be used to inhibit FEAT gene expression. An antisense nucleic acid is a single-stranded nucleic acid sequence (either RNA or DNA) capable of binding to the mRNA or DNA sequence of the FEAT gene. Such an antisense nucleic acid sequence has a length of at least 14 nucleotides, preferably 14 to 100 nucleotides. The antisense nucleic acid binds to the above gene sequence to form a duplex and thereby suppresses the transcription or translation of the FEAT gene.

The antisense nucleic acid may be prepared by chemical or biochemical synthesis procedures known in the art. For example, it is possible to use nucleic acid synthesis procedures using a commonly used DNA synthesizer.

### (3) Decoy nucleic acid

In yet another embodiment of the present invention, a nucleic acid serving as a decoy, which is called a decoy nucleic acid, may be used to inhibit FEAT gene expression.

A decoy nucleic acid is a nucleic acid that suppresses FEAT gene expression through binding to a transcription factor in the FEAT gene or in the FEAT receptor gene to suppress promoter activity, and it is intended to mean a short nucleic acid serving as a decoy that comprises a binding site for the transcription factor. When this nucleic acid is introduced into cancer cells, a transcription factor will bind to this nucleic acid. As a result, the transcription factor will be competitively inhibited from binding to its genomic binding site and thus prevented from being expressed.

Examples of a decoy nucleic acid preferred in the present invention include a nucleic acid binding to a promoter for the FEAT gene, or a nucleic acid binding to a promoter for mRNA of FEAT or for a factor located upstream of FEAT. Such a decoy nucleic acid may be designed as a single or double strand on the basis of the promoter sequence for the FEAT gene. Although the decoy nucleic acid may have any length, its length is 15 to 60 bases, preferably 20 to 30 bases.

The decoy nucleic acid may be either DNA or RNA, and may also include modified nucleic acids.

The decoy nucleic acid to be used in the present invention may be prepared by chemical or biochemical synthesis procedures known in the art. For example, it is possible to use nucleic acid synthesis procedures using a DNA synthesizer commonly used in gene recombination technology. Alternatively, a nucleotide sequence serving as a template may be isolated or synthesized, followed by PCR or gene amplification using a cloning vector. Further, to obtain a decoy nucleic acid which is more stable within cells, bases and other constituents may be modified by chemical modifications such as alkylation, acylation, etc.

It should be noted that in the case of using a decoy nucleic acid, analysis of the promoter's transcriptional activity may be accomplished by using standard assays such as luciferase assay, gel shift assay, RT-PCR, etc. Kits for conducting these assays are also commercially available (e.g., Promega dual luciferase assay kit).

### (4) Aptamer

Furthermore, in the present invention, an aptamer may be used to inhibit FEAT gene expression.

An aptamer is a synthetic DNA or RNA molecule or a peptidic molecule, which has the ability to bind specifically to a target substance, and can be synthesized chemically in a test tube within a short time. Thus, an aptamer may be obtained on the basis of the nucleotide sequence shown in SEQ ID NO: 1, or alternatively, may be obtained by evolutionary engineering procedures known as *in vitro* selection or SELEX techniques.

A nucleic acid aptamer is rapidly degraded and removed from the blood flow by the action of nucleases, and hence it is preferred that the aptamer has optionally been subjected to molecular modification, e.g., with a polyethylene glycol (PEG) chain to thereby extend its half-life.

### (i-2) Substance capable of inhibiting FEAT protein activity

Specific examples of the "substance capable of inhibiting FEAT protein activity" shown in (i) above include substances capable of breaking down the FEAT protein (e.g., various proteases), as well as antagonists of the FEAT protein (e.g., low molecular compounds, hormones, proteins, antibodies and nucleic acids, etc.). In a certain embodiment, such a substance may be an antibody against the FEAT protein. Such an antibody against the FEAT protein is as described above for the test reagent.

### (ii) Immune cells capable of reacting with the FEAT protein

Specific examples of the "immune cells capable of reacting with the FEAT protein" shown in (ii) above include immune cells that recognize the FEAT protein as an antigen. Examples of immune cells include, but are not limited to, helper T cells, cytotoxic T cells (CTLs), natural killer cells, B cells, dendritic cells, macrophages and so on.

Such immune cells may be those transformed by gene recombination to express a gene for an anticancer agent or a gene for a prodrug-converting enzyme that converts a prodrug of the anticancer agent into an active drug.

For example, when the immune cells capable of reacting with the FEAT protein are cells expressing molecules with antitumor activity (hereinafter collectively referred to as "anticancer agents"), such immune cells will contact with tumor cells via the FEAT protein because the FEAT protein is expressed exclusively in tumor cells, and the tumor cells will be killed by the action of the anticancer agents expressed within the immune cells. As used herein, the term "anticancer agents" also includes prodrugs of the anticancer agents.

Examples of anticancer agents include, but are not limited to, parasporin, POGZ, panitumumab, rituximab, cetuximab, trastuzumab, ImmunoMax, bevacizumab, L-asparaginase, dasatinib, nilotinib, erlotinib, sunitinib, sorafenib, lapatinib, axitinib, sunitinib, motesanib, denosumab, nimotuzumab, ibritumomab, tocilizumab, ofatumumab, CS-1008, MORAb-009, MORAb-003, ONO-4538, KW-0761, AMG655, R7159(GA101), R1507, CMC-544, GC33, KRN330m, U3-1287 and so on. The nucleotide sequences of genes encoding these anticancer agents are known.

Alternatively, anticancer agents may also be siRNAs against cancer genes, or shRNAs or dsRNAs from which the siRNAs are produced. Specific examples of cancer genes include the myelocytomatosis oncogene (c-Myc) gene, the v-Src gene, the RAS gene, the B-cell leukemia/lymphoma 2 (BCL2) gene, the phosphatidyl inositol 3-kinase catalytic subunit (PIK3CA) gene, the Cyclin D1 gene, the Liver Kinase B1 (LKB1) gene, the c-Abl gene, the c-Sis gene, the epidermal growth factor receptor (EGFR) gene, the platelet-derived growth factor receptor (PDGFR) gene, the vascular endothelial growth factor receptor (VEGFR) gene, the HER2/neu gene, the Raf kinase gene, the cyclin-dependent kinase genes (e.g., Cyclin dependent kinase 4: Cdk4), and mutant genes thereof. siRNAs are as described above for RNA interference.

When the immune cells of the present invention express a prodrug-converting enzyme, once the above immune cells have been administered to a patient together with a prodrug of an anticancer agent, the above cells will recognize tumor cells or precancerous cells (hereinafter collectively referred to as "tumor cells"), which are expressing the FEAT protein, and will be localized around the tumor cells and will produce the prodrug-converting enzyme exclusively therein. As a result, the administered prodrug will be converted into the original anticancer agent exclusively around the tumor cells, so that the anticancer agent can exert its efficacy exclusively on the tumor cells while minimizing any effects on normal cells.

Examples of such a prodrug-converting enzyme include thymidine kinase, a fusion protein between thymidine kinase and thymidylate kinase, as well as cytosine deaminase and so on. Thymidine kinase is a prodrug-converting enzyme for ganciclovir, a fusion protein between thymidine kinase and thymidylate kinase is a prodrug-converting enzyme for azidothymidine, and cytosine deaminase is a prodrug-converting enzyme for 5-fluorouracil (5-FU). The sequences of genes encoding these prodrug-converting enzymes are known.

Genes for the above anticancer agents (excluding siRNAs against cancer genes) or prodrug-converting enzymes (hereinafter referred to as "anti-cancer genes") may each be inserted as an appropriate expression cassette into an expression vector, and the vector may be used to transform host cells (e.g., immune cells). Such an appropriate expression cassette comprises at least the following as constituent elements:
a promoter that can be transcribed in host cells;
an anti-cancer gene that is linked in-frame to the promoter; and
a sequence that encodes transcription termination and polyadenylation signals for an RNA molecule.

When mammalian immune cells are used as host cells, examples of promoters that can be transcribed in these host cells include, but are not limited to, CMV, CAG, LTR, EF-1α and SV40 promoters, etc.

Expression vectors for siRNAs against cancer genes are as described above for RNA interference.

Such an expression vector may comprise not only the above expression cassette, but also a selection marker expression cassette to select the transformed host cells. Examples of a selection marker include, but are not limited to, positive selection markers (e.g., neomycin resistance gene, hygromycin B phosphotransferase gene), expression reporters (e.g., LacZ, GFP (green fluorescence protein) and luciferase genes), negative selection markers (e.g., herpes simplex virus thymidine kinase gene (HSV-TK), diphtheria toxin A fragment (DTA)), etc.

The transformed host cells can be easily selected by the above markers. For example, in the case of cells carrying the neomycin resistance gene as a marker, these cells may be cultured in a G418-supplemented medium to conduct primary selection. Likewise, when a targeting vector comprises a gene for a fluorescent protein (e.g., GFP) as a marker, drug resistance-based selection may be combined with cell sorting in a FACS (fluorescence activated cell sorter) or the like to isolate fluorescent protein-expressing cells.

Expression vectors which can be used for introduction of anti-cancer genes into host cells are commercially available, and examples include pEGFP-C1™ (Clontech), pCMV-HA™ (Clontech), pMSCVpuro™ (Clontech), pEF-DEST51™ (Invitrogen), pCEP4™ (Invitrogen), ViraPower II Lentiviral Gateway System™ (Invitrogen) and so on. These expression vectors can be introduced into host cells by known gene transfer techniques such as electroporation, microinjection, calcium phosphate transfection, lipofection, virus infection, etc. For details of gene transfer techniques, reference may be made to, e.g., "Sambrook & Russell, Molecular Cloning: A Laboratory Manual Vol. 3, Cold Spring Harbor, Laboratory Press 2001."

Immune cells capable of reacting with the FEAT protein can be prepared in the following manner.

First, the FEAT amino acid sequence is used to predict the amino acid sequence of an immunizing peptide which matches with the histocompatibility antigen (HLA) of each subject, and a peptide having such an amino acid sequence is synthesized. Amino acid sequence prediction for the immunizing peptide which matches with HLA may be accomplished by using the software of BIMAS (http://www-bimas.cit.nih.gov/molbio/hla-bind/) or SYFPEITHI (http://www.syfpeithi.de/Scripts/MHCServer.dll/EpitopePrediction.htm). Then, (1) the above peptide is mixed with Freund's incomplete adjuvant and inoculated as a peptide vaccine into a human subject by the subcutaneous route to produce immune cells in the human body, or alternatively, (2) cells separated from human peripheral blood are cultured together with the peptide and various cytokines to produce immune cells.

### (iii) Substance capable of binding to the FEAT protein, which substance carries an antitumor agent, the substance shown in (i) above or the cells shown in (ii) above

In the present invention, the "substance capable of binding to the FEAT protein, which substance carries an antitumor agent, the substance shown in (i) above or the cells shown in (ii) above" is intended to mean a substance capable of delivering an antitumor agent, the substance shown in (i) above or the cells shown in (ii) above to FEAT-expressing cells. Examples of such a substance include, but are not limited to, peptides, saccharides, small molecules, macromolecules, nucleic acids, metals, antibodies and so on, which are capable of binding to the FEAT protein. In a certain embodiment, such a substance may be an anti-FEAT antibody conjugated with an antitumor agent.

The pharmaceutical composition of the present invention may be administered by any route commonly used for administration of the candidate drug. Specific examples include oral, sublingual, transnasal, transpulmonary, transgastrointestinal and percutaneous routes, eye dropping, intravenous injection, subcutaneous injection, intramuscular injection, intraperitoneal injection, topical injection, and surgical grafting, with intravenous injection being preferred.

The pharmaceutical composition of the present invention may be in a solid dosage form (e.g., capsules, tablets, powders) or may be in a liquid dosage form (e.g., solutions, suspensions or emulsions) or in a semi-liquid dosage form (e.g., ointments, creams or pastes).

When the pharmaceutical composition of the present invention comprises the cells, a suitable dosage form may be exemplified by a cell suspension. Such a cell suspension may comprise a culture medium suitable for survival of the cells, cytokines, serum (preferably autoserum collected from a patient to be administered), nonessential amino acids, antibiotics and so on. On the other hand, when the pharmaceutical composition of the present invention comprises the substance other than the cells, a suitable dosage form may be exemplified by a suspension or a solution. Such a suspension or dosage form may comprise a buffer suitable for maintaining the structure of the above substance (e.g., siRNA, antibody or anticancer agent), physiological saline, protease inhibitors, chelating agents (e.g., EDTA) and so on.

The above active ingredient may be used alone or may be formulated together with other pharmacologically acceptable ingredients. Examples of other pharmacologically acceptable ingredients include, but are not limited to, excipients, binders, disintegrants, antioxidants, preservatives, auxiliaries, lubricants, sweeteners, flavorings and so on.

The pharmaceutical composition of the present invention may comprise the FEAT inhibitor serving as an active ingredient in a therapeutically effective amount. As used herein, the term "therapeutically effective amount" is intended to mean an amount at which the active ingredient can reduce the growth rate of tumor cells or precancerous cells when administered to a subject. For example, in the case of intravenous injections, the therapeutically effective amount is 0.001% to 10% by weight, preferably 0.01 % to 5% by weight, and more preferably 0.1 % to 2% by weight. When the pharmaceutical composition of the present invention comprises the cells, the therapeutically effective amount is 1 × 10³ to 10¹⁰ cells/ml, preferably 1 × 10⁴ to 10⁹ cells/ml, and more preferably 1 × 10⁵ to 10⁸ cells/ml.

The dose and administration frequency of the pharmaceutical composition of the present invention will vary depending on various factors, such as the species, body weight, sex and age of a subject, the severity of tumor disease, the route of administration, etc. However, those skilled in the art including physicians, veterinarians, dentists or pharmacists would be able to determine the dose in consideration of the respective factors. For example, for topical administration to adults (body weight: 60 kg), the pharmaceutical composition of the present invention may be administered daily once to four times, preferably once or twice, and the dose per administration is 0.1 to 150 mg, preferably 1 to 50 mg. When the pharmaceutical composition of the present invention comprises the cells, the dose per administration is 1 × 10³ to 10¹⁰ cells, preferably 1 × 10⁴ to 10⁹ cells, and more preferably 1 × 10⁵ to 10⁸ cells.

For example, a patient may be administered with activated lymphocytes by drip infusion at a dose of 5 × 10⁷ cells or 2 × 10⁸ cells per administration or may be administered with dendritic cells by subcutaneous injection at a dose of 1 × 10⁷ cells, although the dose is not limited thereto.

The numerical values listed above are merely typical ones to describe the therapeutically effective amount, dose and administration frequency; and hence it is highly probable that the increment in the growth rate of tumor cells or precancerous cells will also be reduced even at a value higher or lower than those listed above. Thus, even numerical values higher or lower than those listed above for the therapeutically effective amount, dose and administration frequency also fall within the therapeutically effective amount, dose and administration frequency intended in the pharmaceutical composition of the present invention.

Tumors to be targeted by the pharmaceutical composition of the present invention may be of any type, either benign or malignant. Examples of such tumors include (1) sarcomas such as osteosarcoma and soft tissue sarcoma, (2) cancers such as breast cancer, lung cancer, bladder cancer, kidney cancer, thyroid cancer, prostate cancer, pancreatic cancer, esophageal cancer, gastric cancer, liver cancer, gallbladder cancer, uterine cancer, colorectal cancer, rectal cancer, testicular cancer, uterine cervical cancer, ovarian cancer, skin cancer, tongue cancer and pharynx laryngeal cancer, (3) Hodgkin's and non-Hodgkin's lymphomas, (4) neuroblastoma, (5) melanoma, (6) multiple myeloma, (7) Wilms tumor, (8) leukemias such as acute myelogenous leukemia (AML), chronic myelogenous leukemia (CML), acute lymphocytic leukemia (ALL) and chronic lymphocytic leukemia (CLL), (9) brain tumors such as glioma, (10) retinoblastoma, and so on. Preferred examples include, but are not limited to, tumors which have already been confirmed to express the FEAT protein in humans by Western blotting, immunohistological analysis and/or microarray analysis, as exemplified by ovarian cancer, lung cancer, liver cancer, rectal cancer, uterine cervical cancer, melanoma, testicular cancer, thyroid cancer, uterine cancer, gastric cancer, pharynx laryngeal cancer, breast cancer, prostate cancer, pancreatic cancer, lymphoma, colorectal cancer, bladder cancer, retinoblastoma, etc.

Because of having the ability to suppress tumor growth, the pharmaceutical composition of the present invention can be used not only for tumor prevention, but also as a pharmaceutical composition for tumor treatment. Size reduction of tumor tissues in the patient's body when administered with the pharmaceutical composition of the present invention may be easily traced using a diagnosis instrument such as X-ray, CT, MRI (magnetic resonance imaging), PET (positron emission tomography) or echo.

### 3. Test method

The present invention provides a method for tumor detection, which comprises reacting a test sample taken from a living body with the reagent of the present invention.

The FEAT gene or the FEAT protein is expressed exclusively in tumor cells or precancerous cells, and is not expressed in most normal cells. Thus, if the FEAT gene or the FEAT protein is detected in test cells, such test cells can be diagnosed as tumor cells or precancerous cells.

In the present invention, the living body is not limited in any way and may be of any species of organism. Preferred are mammals including, but not limited to, humans, monkeys, horses, cows, pigs, sheep, dogs, cats, mice, rats, guinea pigs, rabbits, hamsters and so on.

The test sample may be a sample derived from a living body, as exemplified by tissues, cells, mucosa, blood components (serum, plasma, blood cells), lacrimal fluid, nasal discharge, sputum, digestive juice (e.g., saliva, gastric juice, bile, pancreatic juice), urine, feces, sweat, body hair, milk and so on, or alternatively, may be a sample derived from cultured tissues or cells, as exemplified by cultured tissues or cells, extracts of cultured cells, culture supernatants and so on.

Although how to obtain a test sample from a living body will vary depending on the type of the test sample, techniques commonly used for this purpose include blood collection, biopsy, aspiration cytology, urine collection, feces collection, or surgical resection of tumors. Although a cell extract is prepared from the tumor tissue thus obtained, it may be prepared directly from cells contained in the collected tumor (primary tumor cells), or alternatively, may be prepared from cells which are established from the primary tumor cells through one or more cycles of cell division after the cells contained in the collected tumor are cultured under appropriate conditions. The primary tumor cells or the cells established form the primary tumor cells through cell division may be collected and subjected to ultrasonication, treatment with a cell lysing agent, homogenization or the like to obtain a cell extract. The cell extract may be supplemented with a nuclease inhibitor or a protease inhibitor to avoid degradation of nucleic acids or proteins. Moreover, the cell extract may be frozen and stored (preferably at a temperature of -80°C or below) before use in the method of the present invention. Furthermore, the test sample may be a purified protein or nucleic acid obtained from the cell extract prepared as above through a further purification step. As to detailed procedures for cell extract preparation as well as protein and nucleic acid purification, reference may be made to the following: "Sambrook & Russell, Molecular Cloning: A Laboratory Manual Vol. 3, Cold Spring Harbor Laboratory Press 2001," "Ausubel, Current Protocols in Molecular Biology, John Wiley & Sons 1987-1997."

In this detection method, to react the test sample with the test reagent of the present invention, these sample and reagent may be mixed together. After mixing, the mixture is preferably shaken (e.g., with a shaker or a rotor) under gentle conditions to ensure a uniform distribution of components in the mixture. The method of the present invention is performed at atmospheric temperature (around 25°C) or below, preferably at 15°C, 10°C, 8°C, 5°C or below, and most preferably at 4 ± 1°C, in terms of avoiding degradation of the FEAT protein or the FEAT gene contained in the sample and the nucleic acid or antibody contained in the reagent of the present invention.

Moreover, prior to the reaction between the test sample and the reagent of the present invention, either or both of the test sample and the reagent of the present invention may be blocked by being mixed with a blocking agent. Alternatively, the reaction may be performed in a mixture in which the test sample, the reagent of the present invention and the blocking agent are all mixed together. Examples of such a blocking agent include, but are not limited to, serum-containing buffers or skimmed milk-containing buffers.

Assay methods for detection of the FEAT gene or protein are as described above for the reagent for tumor detection.

### 4. Tumor animal model

The inventors of the present invention have prepared a transgenic (Tg) animal overexpressing the FEAT protein by introduction of the FEAT gene, and have found that FEAT protein overexpression causes the Tg animal to form tumors in a plurality of organs. Tumors formed in the Tg animal overlap with tumors in human patients in their pathological features and genetic changes. Thus, the Tg animal is useful as a tumor model reflecting human tumors in detail.

Accordingly, the present invention provides a tumor animal model, which consists of a transgenic non-human animal transformed with the FEAT gene.

In the present invention, the "FEAT gene" is as described above for the test reagent.

The "transgenic non-human animal" intended in the present invention is not limited in any way as long as it is a non-human animal. Preferred are mammals (e.g., horses, cows, pigs, dogs, monkeys, mice, rats, guinea pigs, rabbits), and more preferred are rodents such as mice, rats, guinea pigs, rabbits and the like in terms of ease of obtaining next generation animals by gene recombination and crossing.

### 4-1. Procedures for transgenic animal preparation

An FEAT gene expression vector is prepared and introduced into fertilized eggs. The type of expression vector, how to introduce it into host cells, and how to select a transformant are as described above for the pharmaceutical composition.

Introduction into fertilized eggs may be accomplished by using microinjection (Hogan, B. et al. "Manipulating the Mouse Embryo" Cold Spring Harbor Laboratory Press, 1988).

Microinjection is a technique for direct injection of an FEAT gene expression vector into fertilized eggs. In this technique, fertilized eggs collected from animals are directly injected with an FEAT gene expression vector using a micromanipulator or the like under a microscope.

The fertilized eggs thus prepared may be implanted into and allowed to develop in the uteri of foster mothers (pseudopregnant animals) to thereby obtain a desired transgenic animal.

The above transgenic animal may further be crossed with a wild-type animal of the same line or with another transgenic animal for maintenance of the transgenic animal line. The genotype of each animal can be determined by genotype analysis based on Southern blotting or PCR as described above.

Progeny animals of the FEAT gene transgenic animal prepared as above also fall within the animal model of the present invention as long as the FEAT gene is overexpressed.

The animal model of the present invention would be useful as a model for various types of tumors when prepared by varying the type or line of animal. For instance, examples of tumors include (1) sarcomas such as osteosarcoma and soft tissue sarcoma, (2) cancers such as breast cancer, lung cancer, bladder cancer, kidney cancer, thyroid cancer, prostate cancer, pancreatic cancer, esophageal cancer, gastric cancer, liver cancer, gallbladder cancer, uterine cancer, colorectal cancer, rectal cancer, testicular cancer, uterine cervical cancer, ovarian cancer, skin cancer, tongue cancer and pharynx laryngeal cancer, (3) Hodgkin's and non-Hodgkin's lymphomas, (4) neuroblastoma, (5) melanoma, (6) multiple myeloma, (7) Wilms tumor, (8) leukemias such as acute myelogenous leukemia (AML), chronic myelogenous leukemia (CML), acute lymphocytic leukemia (ALL) and chronic lymphocytic leukemia (CLL), (9) brain tumors such as glioma, (10) retinoblastoma, and so on. Preferred examples include, but are not limited to, tumors which have already been confirmed to express the FEAT protein in humans by Western blotting, immunohistological analysis and/or microarray analysis, as exemplified by ovarian cancer, lung cancer, liver cancer, rectal cancer, uterine cervical cancer, melanoma, testicular cancer, thyroid cancer, uterine cancer, gastric cancer, pharynx laryngeal cancer, breast cancer, prostate cancer, pancreatic cancer, lymphoma, colorectal cancer, bladder cancer, retinoblastoma, etc. The animal model of the present invention is preferably a transgenic mouse.

The animal model of the present invention is particularly useful as an animal model for hepatocellular carcinoma (HCC) and/or malignant lymphoma.

### EXAMPLES

The present invention will be further described in more detail by way of the following illustrative examples, although the present invention is no limited to the embodiments disclosed herein.

### <1. Experimental procedures>

### Reagents

Agonistic anti-Fas IgM monoclonal antibody (CH-11) was purchased from MBL (Nagoya, Japan). Benzyloxycarbonyl-Val-Ala-Asp(OMe)-fluoromethylketone (zVAD-fmk) for use as a caspase inhibitor was purchased from Enzyme Systems (Dublin, California, USA).

### Plasmids

His-tagged DNA fragmentation factor (DFF) 40 and DFF 45 (Liu, X. (1999) J. Biol. Chem. 274, 13836-13840) were kindly provided by Dr. Xiaodong Wang (Howard Hughes Medical Institute and Department of Biochemistry, University of Texas Southwestern Medical Center, Dallas, Texas). Plasmid pcDL-SRα-procaspase-3 was kindly provided by Dr. Fumiko Toyoshima-Morimoto (Institute for Virus Research, Kyoto University, Kyoto, Japan).

### Purification of recombinant caspases

His-tagged recombinant caspase-3 and caspase-6 encoded by pET-16b plasmids (Novagen, Merck KgaA, Darmstadt, Germany) were purified from E. *coli* BL21(DE3)pLys cells (Novagen, Merck KGaA) in the following manner.

After 1 mM isopropyl-β-D-thiogalactopyranoside (IPTG) was added to induce caspase expression for 1 hour, the E. *coli* cells were washed with phosphate-buffered saline (PBS) and then lysed by ultrasonication in a lysis buffer (containing 50 mM Tris-HCl (pH 8.0), 0.5 mM sucrose, 5% glycerol, 1 mM phenylmethylsulfonyl fluoride (PMSF), 1% (v/v) aprotinin (Sigma, Saint Louis, MO), and 1 µg/ml CLAP (chymotrypsin, leupeptin, antipain and pepstatin)). Subsequently, the following step was performed at 4°C.

The cell lysate was centrifuged, and the supernatant was supplemented with 10 mM β-mercaptoethanol and 5 mM imidazole. This mixture was applied to a Ni²⁺-NTA agarose column (Qiagen, Hilden, Germany) which had been equilibrated with the lysis buffer. The column was then washed well with a washing buffer (50 mM Tris-HCl (pH 8.0), 0.5 mM sucrose, 5% glycerol, 10 mM β-mercaptoethanol, 60 mM imidazole, 400 mM NaCl, 0.04% (v/v) Nonidet P-40 (NP-40)). His-tagged caspases were eluted with an elution buffer (50 mM Tris-HCl (pH 8.0), 0.5 mM sucrose, 5% glycerol, 500 mM imidazole, 1 mM PMSF, 1% (v/v) aprotinin, 1 µg/ml CLAP) and then dialyzed against KPM buffer (50 mM KCl, 50 mM PIPES, pH 7.0, 10 mM EGTA, 1.92 mM MgCl₂, 1 mM dithiothreitol (DTT), 20 µM cytochalasin B, 100 µM PMSF, 1 µg/ml CLAP).

### Purification of rat-derived FEAT protein

Rat liver cytoplasmic extracts (4 g each of the supernatants centrifuged at 100,000 × g from six Wister rats) were purified sequentially by cation exchange chromatography (SP-Sepharose FF; GE Healthcare Bio-Sciences), ammonium sulfate fractionation, hydrophobic interaction chromatography (Phenyl Sepharose HP; GE Healthcare Bio-Sciences), ammonium sulfate fractionation, anion exchange chromatography (DE52; Whatman, GE Healthcare Bio-Sciences), metal chelate chromatography (zinc) (HiTrap chelating, FPLC; GE Healthcare Bio-Sciences) and gel filtration (Superdex 200, FPLC; GE Healthcare Bio-Sciences).

The fractions obtained in the above respective purification steps were each applied to a desalting column (Econo-Pac 10DG column or Bio-Spin chromatographic column; Bio-Rad Laboratories, Inc., Hercules, CA) to replace the solvent with KPM buffer. Each fraction was incubated at 37°C for 2 hours together with His-tagged caspase-3 or caspase-6, isolated nuclei (Lazebnik, Y. A. et al (1993) J. Cell Biol. 123, 7-22) and an ATP regeneration system (Takahashi, A. et al (1997) Exp. Cell Res. 231, 123-131). The nuclei were stained with 1 µg/ml 4',6-diamidino-2-phenylindole (DAPI) and observed for their morphology under a fluorescence microscope (Olympus, Tokyo, Japan).

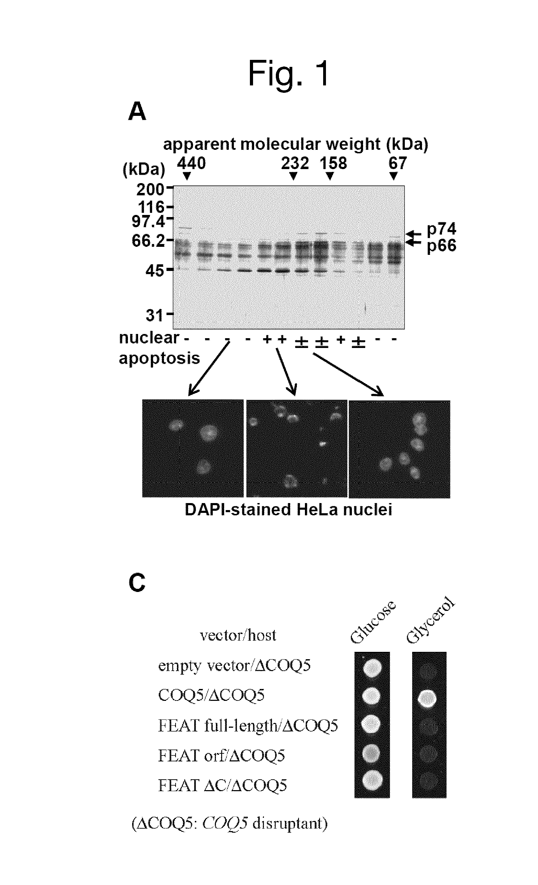

In addition, proteins contained in each fraction were separated by sodium dodecyl sulfate-polyacrylamide electrophoresis (SDS-PAGE) (BIO CRAFT, Tokyo, Japan) and stained with a 2D silver staining kit II (Daiichi Pure Chemicals, Tokyo, Japan) (Figure 1A, upper panel).

The purified proteins were separated by SDS-PAGE and transferred onto a polyvinylidene difluoride (PVDF) membrane (ProBlott; Applied Biosystems, Lincoln Centre Drive Foster City, California), followed by staining with 0.2% Ponceau S and 1% acetic acid. The membrane was excised and digested with trypsin, followed by mass spectrometry. The proteins were not able to be collected in an amount sufficient for microsequencing. To increase protein yields, the above purification steps were modified as follows:
cation exchange chromatography (SP-Sepharose FF), ammonium sulfate fractionation, anion exchange chromatography (DE52), further cation exchange chromatography (SP-Sepharose HP, FPLC; GE Healthcare Bio-Sciences), followed by gel filtration (Superdex 200, FPLC). With the purification steps thus modified, the purified proteins were able to be microsequenced as a number of internal oligopeptides.

### Cloning of human FEAT cDNA

RNAs were collected from Jurkat T cells, KB epithelial cancer cells and HeLa cells. Reverse transcriptase PCR reaction (RT-PCR) was performed with primers prepared based on the sequence of an expressed sequence tag (EST) for human FEAT. A 2.5 kb cDNA fragment detected in all of the cell lines was subcloned and sequenced, and then used as a [³²P]-labeled probe in screening against the human skin λZAP II cDNA library (Stratagene, La Jolla, CA) (Takahashi, K. et al (1994) J. Cell Biol. 127, 505-520) in accordance with the manufacturer's instructions.

### Preparation of antibodies

A His-tagged FEAT amino-terminal (N-terminal) protein (amino acid (aa) sequence at positions 1-274; His-FEATΔC) and a His-tagged carboxyl-terminal (C-terminal) protein (amino acid sequence at positions 288-699; His-FEATΔN) encoded by pET-16b plasmids (Novagen, Merck KGaA) were allowed to be expressed in E. *coli* BL21(DE3)pLys cells (Novagen, Merck KGaA). The expressed proteins were purified using Ni²⁺-NTA columns (Qiagen) in accordance with the manufacturer's instructions under denaturing conditions. The purified proteins were diluted with PBS and injected into rabbits. The rabbit antisera were affinity-purified with the His-FEATΔC protein and the His-FEATΔN protein (Operon Biotechnologies, Tokyo, Japan).

### Transfection

An expression plasmid for mammalian cells was transfected into COS-7, 293, HeLa or MCF-7 cells using FuGENE 6 (Roche, Indianapolis, Indiana). Likewise, the same plasmid was also transfected into Karpas 299 cells using DMRIE-C (Roche). Transfection was performed in accordance with the instructions of the transfection reagent manufacturer.

### Immunoprecipitation

The following steps were all performed at 4°C. Cells were lysed over 15 minutes in TNE buffer (10 mM Tris-HCl (pH 7.8), 150 mM NaCl, 1% NP-40, 1 mM EDTA) supplemented with 1 mM PMSF, 1% (v/v) aprotinin and 1 µg/ml CLAP, and then centrifuged at 15000 rpm for 30 minutes. To the supernatant, each antibody was added and incubated for 30 minutes. Protein G Sepharose beads (GE Healthcare Bio-Sciences) equilibrated with TNE buffer were added, and the resulting mixture was rotated for 2 hours. The beads were washed three times with TNE buffer. The beads were incubated in a sample buffer for SDS-PAGE at 95°C for 3 minutes to elute proteins bound to the beads.

### Methyltransferase activity assay

Plasmids pcDL-SRα-myc and pcDL-SRα-HA were used to overexpress Myc-tagged or HA-tagged human FEAT, FEATΔC and FEATΔN in COS-7 cells, followed by immunoprecipitation with anti-human c-Myc monoclonal antibody (9E10; sc-40: Santa Cruz Biotechnology, Inc., Santa Cruz, CA) and anti-HA monoclonal antibody (Y-11; sc-805: Santa Cruz Biotechnology, Inc., Santa Cruz, CA). The immunoprecipitates were equilibrated with a methyltransferase buffer (25 mM Tris-HCl, pH 7.5, 150 mM NaCl, 1 mM EDTA, 1 mM EGTA). *E. coli* BL21(DE3)pLys cells carrying pET-16b plasmids encoding His-tagged FEAT and FEAT mutants were incubated in the presence of 1 mM IPTG at 16°C for 16 hours to induce expression, and the expressed proteins were purified using Ni²⁺-NTA columns (Qiagen) in accordance with the manufacturer's instructions under denaturing conditions. The purified proteins were then dialyzed against a dialysis buffer (50 mM phosphate buffer (pH 7.4), 50 mM NaCl, 10% glycerol, 5 mM DTT).

From the peripheral blood of healthy adult volunteers, mononuclear cells were isolated with a Percoll (GE Healthcare Bio-Sciences), as previously reported (Takahashi, A. et al (1992) Eur. J. Haematol. 48, 196-201). Jurkat cells, Karpas 299 cells and human peripheral blood mononuclear cells were each lysed with a lysis buffer (25 mM Tris-HCl (pH 7.5), 0.1% NP-40, 1 mM EDTA, 1 mM EGTA, 1 mM PMSF, 1% (v/v) aprotinin, 1 µg/ml CLAP) and used as a substrate. Recombinant FEAT (80 ng/µl, immunoprecipitated FEAT or mutants thereof were each mixed with the cell lysate (containing 4 µg protein per 1 µl) and 5 nCi/µl [¹⁴C]-S-adenosylmethionine in the methyltransferase buffer and incubated at 37°C for 60 minutes. The radiolabeled methylated proteins were separated by 12.5% SDS-PAGE and visualized with an image analyzer (Fuji Photo Film Co., Ltd., Tokyo, Japan).

### Spermine/spermidine synthase activity

*E. coli* BL21(DE3)pLys cells were treated with 1 mM IPTG at 37°C for 4 hours or 16 hours to induce expression of His-tagged FEAT or mutants thereof encoded by pET-16b plasmid. To determine the amounts of spermine and spermidine produced in the *E. coli* cells, the spermine/putrescine ratio and the spermidine/putrescine ratio were measured. For measurement of *in vitro* spermine and spermidine synthase activity, recombinant FEAT (30 µg/ml), immunoprecipitated FEAT or mutants thereof were each mixed with a reaction buffer (100 mM Tris-HCl (pH 7.5), 5 mM DTT, 1 mM EDTA, 1 mM EGTA) supplemented with 1 mM putrescine dihydrochloride (Wako Pure Chemical Industries, Osaka, Japan) and 0.2 mM decarboxylated S-adenosylmethionine (provided by Dr. Akira Shirahata, Department of Pharmaceutical Sciences, Josai University, Saitama, Japan), and then incubated at 37°C for 30 minutes. 5% (w/v) Trichloroacetic acid was added to stop the reaction. The samples were centrifuged at 14000 rpm at 4°C for 10 minutes, and the supernatants were analyzed for spermine and spermidine. As a positive control, *Nicotiana sylyvestris*-derived spermidine synthetase (whose cDNA was provided by Dr. Takashi Hashimoto, Division of Molecular Biology, Nara Institute of Science and Technology, Nara, Japan) was used.

### Complementation test of ubiquinone biosynthesis in yeast

Having an SAM-binding motif at the N-terminal end is a feature also found in ubiquinone synthetase. Thus, the 2.6 kb full-length cDNA or open reading frame (ORF) encoding human FEAT or cDNA of FEATΔC was subcloned into pENTR3C vector (Invitrogen Corp., Carlsbad, California). LR recombination was conducted with pDR196GW vector (provided by Dr. W. Frommer, Carnegie Institution, Washington DC), in which reading frame cassette A in a Gateway cloning system was integrated into the SmaI site of yeast expression plasmid pDR196 (Rentsch, D. et al (1995) FEBS Lett. 370, 264-268), to thereby prepare pDR196GW-FEAT(full-length), pDR196GW-FEAT(ORF) and pDR196GW-FEATAC plasmids. These plasmids encode FEAT or FEATΔC under the control of the plasma membrane ATPase 1 promoter which allows strong constitutive gene expression in yeast. These plasmids were each introduced into budding yeast (*Saccharomyces cerevisiae*) strain W303-1A-Δcoq5 (provided by Dr. C. F. Clarke and Dr. P. Gin, University of California, Los Angeles, California). In this strain, the COQ5 gene is disrupted (Barkovich, R.J. et al (1997) J. Biol. Chem. 272, 9182-9188).

The yeast strains carrying the above FEAT expression vectors were each cultured in SD (-uracil) liquid medium until they reached the mid-log phase (OD₆₀₀ about 2.0). Then, the cultured products (5 µl each) were spotted onto SD (-uracil) agar plates containing minimal medium supplemented with either glucose (control) or glycerol as a sole carbon source. Since ubiquinone is required to use nonfermentable carbon sources, the budding yeast strain ΔCOQ5 which lacks C-methyltransferase activity shows growth defect on a glycerol plate. These plates were incubated at 30°C for 24 hours (glucose) or 48 hours (glycerol), and recovery of the growth ability on the glycerol plates was used as an indicator to evaluate complementation for the COQ5 gene.

For use as a positive control in the above complementation test, yeast expressing the COQ5 gene was prepared in the following manner. Using an RNeasy Plant Mini kit (Qiagen), total RNA was extracted from the wild-type budding yeast (W303-1A strain). The total RNA (2.0 µg) and Superscript III RNase H⁻ (Invitrogen) were used to conduct reverse transcription reaction. The resulting cDNA, KOD-Plus-DNA polymerase (TOYOBO Co., Ltd., Osaka, Japan) and the primers shown below were used in PCR to amplify the coding region of the COQ5 gene.
COQ5 forward: 5'-GGGGTACCATGTTGATTTCTTCACGGATCGTTC-3' (SEQ ID NO: 9)
COQ5 reverse: 5'-GCGCTCGAGTTAAACTTTAATGCCCCAATGGAT-3' (SEQ ID NO: 10)

The underlined sequences in the above primers are KpnI and XhoI recognition sites, respectively. The resulting PCT product (924 bp) was subcloned into pENTR3C vector to prepare pENTR3C-COQ5. Then, pENTR3C-COQ5 was used for LR recombination with pDR196GW to prepare pDR196GW-COQ5.

### Western blotting

Cells were fixed by being soaked in ice-cold 10% (w/v) trichloroacetic acid/PBS for 30 minutes on ice. After centrifugation at 15000 rpm at 37°C for 5 minutes, the pellet was suspended again in an SDS-PAGE sample buffer supplemented with 150 mM Tris-HCl (pH 8.8), and then incubated at 95°C for 3 minutes. The following steps were all performed at room temperature.

The proteins separated by SDS-PAGE were transferred onto an Immobilon-P PVDF membrane (Millipore, Billerica, Massachusetts) using an NA-1512 semidry electric transfer apparatuses (Nihon Eido, Tokyo, Japan) and a transfer buffer (175 mM Tris, 1.344 M glycine, and 5% methanol) at 180 mA over 45 minutes. The membrane was blocked for 15 minutes with a blocking buffer (PBS, 0.1% Tween-20 (Bio-Rad), 5% skimmed milk (Difco Skim Milk; Difco Laboratories, BD Biosciences, San Jose, California)) and then incubated for 45 minutes with a blocking buffer containing a primary antibody for staining. After washing three times with PBS/0.1% Tween-20 (PBS-Tween) for 5 minutes, the membrane was probed by being incubated for 15 minutes with a horseradish peroxidase-conjugated secondary antibody (sheep-derived anti-rabbit IgG (sc-2004: Santa Cruz Biotechnology, Inc., Santa Cruz, California) or anti-mouse IgG (sc-2005: Santa Cruz Biotechnology)) which had been diluted 1:10000 with the blocking buffer. The membrane was washed four times with PBS-Tween for 5 minutes and then visualized with an ECL detection system (GE Healthcare Bio-Sciences).

The primary antibodies used are as follows: anti-actin antibody (A5060; Sigma) and anti-human c-Myc antibody (Anti-Myc Tag, 06-549; Upstate, Millipore).

An INSTA-Blot membrane (IMB-105) (IMGENEX, San Diego, California) contains cell lysates of the following human cell lines (10 µg protein per lane): HeLa (uterine cervical cancer), Jurkat (T-cell leukemia), Daudi (Burkitt's lymphoma), 293 (fetal kidney cells transformed with adenovirus type 5), Rh 30 (rhabdomyosarcoma), A375 (malignant melanoma), T98G (glioblastoma), HCT-116 (colorectal cancer) and Hep-2 (pharyngeal cancer). Multiple Tissue Blot (human) (WB46) and Human Tumor Tissue Blot (WB51) were purchased from Calbiochem (Merck KGaA). WB46 contains proteins derived from various normal tissues (75 µg protein per lane). WB51 contains proteins derived from the following cancer cells (50 µg protein per lane): ovary stromal sarcoma, lung adenocarcinoma, hepatocellular carcinoma, rectal adenocarcinoma, cervical squamous cell carcinoma, skin malignant melanoma, testicular embryonal carcinoma, follicular thyroid cancer, uterine adenocarcinoma, gastric adenocarcinoma, laryngopharyngeal squamous cell carcinoma, breast ductal carcinoma, benign prostatic hyperplasia, and pancreatic adenocarcinoma.

### Determination of caspase cleavage sites

Caspase is generally known to cleave the carboxyl side of aspartic acid residues in its substrate (Earnshaw, W.C. et al (1999) Annu. Rev. Biochem. 68, 383-424). Candidate aspartic acids as cleavage sites encoded by FEAT cDNA inserted into pBluescript SK(-) plasmid (Stratagene) were each mutated to alanine using a GeneEditor in vitro Site-Directed Mutagenesis System (Promega, Madison, Wisconsin). *In vitro* transcription/translation of the cDNA was conducted using a TNT T7/T3 Coupled Reticulocyte Lysate System (Promega) and a Tran³⁵S-label (MP Biomedicals, Irvine, California). The [³⁵S]-labeled recombinant wild-type or mutant FEAT proteins were incubated at 37°C for 2 hours together with purified recombinant caspase-3, and the products were separated by SDS-PAGE and visualized by autoradiography.

### RT-PCR analysis of malignant lymphoma cell lines

The following cell lines have been established from malignant lymphoma patients (Akasaka et al., 1996): Satoh (endemic Burkitt's lymphoma cell line), KS-Bu3 (sporadic Burkitt's lymphoma cell line), DL-4 (DLBL cell line with t(8;14) translocation), KIS-1 (DLBL cell line with t(9;14) chromosomal translocation), Yoshikawa (testicular DLBL cell line), Muranaka (T-cell lymphoma cell line), Karpas (ALCL cell line with t(2;5) chromosomal translocation), DLBL (diffuse large B-cell lymphoma), and ALCL (anaplastic large cell lymphoma).

TRIzol (Invitrogen) was used to isolate total RNA. Superscript II reverse transcriptase (Invitrogen) and an oligo (dT) primer were used to synthesize the first strand of cDNA. KOD Dash DNA polymerase (TOYOBO) and the following primers were used to perform PCR reaction:
FEAT forward: 5'-AAAGTGTGAGGGGCTCTTCA-3' (SEQ ID NO: 11)
FEAT reverse: 5'-CCCTAATCTTCAGGCAACCT-3' (SEQ ID NO: 12)
β-actin forward: 5'-TCCTGTGGCATCCACGAAACT-3' (SEQ ID NO: 13)
β-actin reverse: 5'-GAAGCATTTGCGGTGGACGAT-3' (SEQ ID NO: 14)

### Analysis of apoptotic DNA cleavage in cells expressing enhanced green fluorescent protein (EGFP)

Cells were co-transfected with a plasmid encoding wild-type FEAT or a mutant FEAT protein and with pEGFP (Clontech Laboratories, Inc., Takara Bio Inc), followed by treatment with staurosporine (STS) (Sigma). EGFP-expressing apoptotic cells with fragmented DNA were quantified as described in Lamm et al., 1997. Namely, the cells were fixed with a mild fixing solution (2% paraformaldehyde, 100 mM NaCl, 300 mM sucrose, 3 mM MgCl₂, 1 mM EGTA, 10 mM PIPES (pH 7.0)) at room temperature for 30 minutes and then fixed with ethanol, followed by staining with propidium iodide (PI: Calbiochem, Merck KGaA) and flow cytometry analysis.

### Neutrophil isolation, protein introduction and spontaneous apoptosis analysis

As described in Takahashi, A. et al. ((1992) Eur. J. Haematol. 48, 196-201), two-step Percoll gradient separation (GE Healthcare Bio-Sciences) was performed to isolate neutrophils from the peripheral blood of healthy adult volunteers. The PTD-4 sequence (YARAAARQARA: SEQ ID NO: 15) (Ho, A. et al (2001) Cancer Res. 61, 474-477), which makes a protein permeable across the cell membrane, was introduced between a His tag and the N-terminal end of wild-type or mutant FEAT protein. The neutrophils were suspended in RPMI-1640 (Sigma) medium supplemented with 10% fetal bovine serum (FBS) (GIBCO, Invitrogen), 50 units/ml penicillin and 50 µg/ml streptomycin (GIBCO, Invitrogen) (FBS-RPMI), and then treated with 1 µM purified protein at 37°C for 15 minutes. Then, the cells were diluted with FBS-RPMI and incubated at 37°C for 24 hours. The cells were stained with FITC-conjugated annexin V and PI (ApoAlert annexin V Apoptosis Detection kit, Clontech Laboratories, Takara Bio), and the amount of cells with fragmented DNA was analyzed by flow cytometry to determine the apoptosis ratio of the neutrophils.

### RNA interference (RNAi)

pMuniH1 plasmid was kindly provided by Dr. Arasambattu K. Munirajan (University of Madras, Teramani, India). The pMuniH1 plasmid encodes short hairpin RNA (shRNA), whose expression is driven by the H1 RNA promoter, and a neomycin selection marker. In accordance with the criteria described in Ui-Tei, K. et al. ((2004) Nucleic Acids Res. 32, 936-948), sites appropriate for RNAi were selected from FEAT cDNA. As a result of selection, oligonucleotides targeting 21 base pairs starting from positions 183, 259, 1385, 1550, 1634 and 2056 in the ORF were each synthesized and ligated to pMuniH1. The shRNA plasmids were co-transfected with a plasmid encoding Myc-tagged FEAT into HeLa cells, and the ability of each plasmid to suppress myc-FEAT expression was evaluated by Western blotting with anti-human c-Myc polyclonal antibody (anti-Myc tag). Plasmids with RNAi activity were selected and transfected into HeLa cells. After selection for 10 days in the presence of 400 µg/ml G418 (Sigma), colonies were picked up and screened for clones in which endogenous FEAT protein expression was suppressed.

### Immunofluorescence assay

Unless otherwise specified, the following steps were all performed at room temperature. Cells adhered onto 35 mm glass bottom dishes (Matsunami Glass, Osaka, Japan) were fixed with 3.7% formaldehyde/PBS for 10 minutes, permeabilized with 0.5% (w/w) Triton X-100/PBS for 30 minutes, and then blocked with 3% bovine serum albumin (BSA)/PBS. After washing with PBS, the cells were incubated for 1 hour with an anti-FEATΔN antibody (having minimal cross-reactivity with other proteins: see Figures 4A and 5A) which had been diluted with 3% BSA-PBS. The cells were washed with PBS and incubated for 1 hour with Alexa Fluor 488- or 588-conjugated anti-rabbit antibody (Molecular Probes, Invitrogen) and 1 µg/ml DAPI. After washing with PBS, a cover slip was placed on each glass bottom dish and mounted with a Prolong Gold Antifade Reagent (Molecular Probes, Invitrogen). The stained cells were observed under a DM IRE2 fluorescence microscope (Leica Microsystems, Wetzlar, Germany) and imaged using a DC 350F camera (Leica Microsystems) controllable by Leica Q Fluoro software (Leica Microsystems Imaging Solutions, Cambridge, UK). For visualization of actin, Alexa Fluor 488-conjugated phalloidin (Molecular Probes, Invitrogen) was added together with the secondary antibody. For visualization of mitochondria, the living cells were incubated with Mitotracker Red (Molecular Probes, Invitrogen) at 37°C for 30 minutes before being fixed. For visualization of endoplasmic reticula (ERs), the cells were fixed with 4% paraformaldehyde/PBS for 15 minutes, permeabilized with 0.1% Triton X-100/PBS for 10 minutes, and then incubated with an ER staining reagent (Subcellular Structure Localization Kit; Chemicon International, Millipore) at 37°C for 30 minutes. Subsequently, blocking was performed and FEAT staining was further performed.

### Gene expression profiling with microarrays

The cDNA of human FEAT ORF was subcloned into entry vector pENTR3C in a Gateway cloning system (Invitrogen). LR recombination was conducted with pEF-DEST51 vector (Invitrogen) to prepare pEF-DEST51-FEAT which drives FEAT expression under the control of the elongation factor 1α promoter. Using HilyMax (Dojindo Laboratories, Kumamoto, Japan), pEF-DEST51-FEAT was transfected into NIH3T3 cells. After selection for 7 days in the presence of 10 µg/ml blasticidin S (Kaken Pharmaceutical Co., Ltd., Tokyo, Japan), colonies were picked up and screened for clones in which the FEAT protein was overexpressed. The ccdB gene in pEF-DEST51 vector was removed to prepare pEF-DEST51-ΔccdB plasmid. The pEF-DEST51-ΔccdB plasmid was stably transfected into NIH3T3 cells to prepare control cell lines ΔccdB-1, -2 and -3.

Using an RNeasy Mini kit (Invitrogen), total RNA was extracted from the control cells and the NIH3T3 cell lines forced to express FEAT (FEAT-3, -15 and -20). Gene expression profiles were analyzed with a Sentrix Mouse WG-6 v2 BeadChip Array (Illumina Inc., San Diego, CA) at the Research Support Center, Graduate School of Medical Sciences, Kyushu University. First, BeadStudio 3.0 software was used to normalize the raw data of gene expression. Average signals with a detection p-value >0.01 (based on Illumina replicate gene probes) were excluded from the analysis. Gene set enrichment analysis (GSEA) was performed using software available from the following URL.
http://www.broadinstitute.org/gsea/

### Preparation of FEAT transgenic (Tg) mice

All animal experiments were conducted under the approval of the Animal Experiment Committee of the Chiba Cancer Center. The cDNA of human FEAT ORF was inserted into the BamHI site of pKCR-H-2K_{d} vector (Nishi, M. et al (1988) Nature 331, 267-269) (provided by Dr. T. Honjo and Dr. I. Chung-Okazaki (Kyoto University, Kyoto, Japan)). As described in Nagy, A., Gertsenstein, M., Vintersten, C., and Behringer, R. eds. ((2003). Manipulating the Mouse Embryo: A Laboratory Manual., 3rd Edition (CSH Laboratory Press)), a KpnI/SphI fragment containing H-2Kd (MHC class I) promoter, FEAT ORF, β-globin intron and SV40 poly(A) signal was purified.

Tg mice and littermate transgene-negative (non-transgenic: non-Tg) mice were maintained in an SPF (specific pathogen free) animal facility at the Chiba Cancer Center Research Institute. Few progeny mice were born from female Tg mice, and hence attempts to prepare homozygous Tg mice ended in failure. For this reason, male Tg mice were crossed with normal female C57BL/6 mice (Charles River Laboratories, Inc., Wilmington, Massachusetts) to maintain Tg mouse lines. The mice were tested at fixed intervals for signs of tumor and other diseases. Mice which developed tumors or were in a moribund state were euthanized and necropsied. Sperm were collected from the epididymis of each Tg mouse and their cryopreservation was entrusted to Kyudo Co., Ltd. (Tosu, Japan).

### Protein expression in mouse organs

Tg mice and non-Tg littermates at 6 months of age were euthanized with carbon dioxide and then dissected. Each organ was frozen in liquid nitrogen and fragmented with a hammer. The fragmented tissues were suspended in RIPA buffer (50 mM Tris-HCl (pH 7.4), 150 mM NaCl, 1% (v/v) NP-40, 1% deoxycholate (Sigma), 0.05% SDS) and completely lysed by repeating three rounds of freezing in liquid nitrogen and thawing on ice. The cell lysates were centrifuged at 15000 rpm at 0°C for 10 minutes to collect the supernatants. The protein concentration in the extracts was determined using a Bio-Rad Protein Assay kit (Bio-Rad) and the samples were loaded on an SDS-PAGE minigel in a total protein amount of 30 µg per lane, followed by Western blotting with an anti-FEATΔN antibody.

### Apoptotic cell death in mouse thymocytes

The thymus was excised and homogenized at 37°C using a 1 ml sterilized syringe together with 10% FBS-supplemented RPMI-1640 medium (Sigma) to isolate thymocytes. In a 5% CO₂ incubator, the cells were then treated with Fas ligand (rhsSuperFasLigand: Alexis, Lausen, Switzerland) or dexamethasone (Nacalai Tesque, Kyoto, Japan) or were maintained untreated. The cells were stained with annexin V-CFS (R&D Systems, Inc., Minneapolis, Minnesota) and PI, followed by flow cytometry analysis to determine cell death.

### Microscopic analysis of mouse tissues

The mouse organs were each excised, fixed with 3.7% formaldehyde/PBS, sliced into 2 mm sections, placed into tissue cassettes (Tissue-Tek Uni-Cassette; Sakura Finetek Japan Co., Ltd., Tokyo, Japan) and then immersed in fresh fixative. Further processing and histopathological studies were performed in Narabyouri Research Co., Ltd. (Nara, Japan).

The fixed tissues were dehydrated with serial dilutions of ethanol (20% to 100%) and a xylene solution. The tissues were each embedded in paraffin using an automatic tissue processor, sliced with a microtome, deparaffinized and rehydrated, followed by staining with hematoxylin and eosin (H&E). Using an Autostainer (DAKO, Glostrup, Denmark), immunohistochemical staining was performed for the following markers: CD45R (B220) (B cell marker) and CD3 (T cell marker).

### Immunohistochemistry

Paraffin-embedded sections were deparaffinized with a Clear-Advantage (Polysciences, Inc., Warrington, Pennsylvania), rehydrated, treated with a Citrate-based Antigen Unmasking Solution (Vector Laboratories Inc., Burlingame, California), and stained using an ImmPRESS kit (Vector Laboratories) and an ImmPACT Chromogen (Vector Laboratories) in accordance with the manufacturer's instructions. The slides were counterstained with Mayer's Hematoxylin (Merck KGaA), and cover slips were mounted with a Gel/Mount aqueous mounting medium (Biomeda, Corp., Foster City, California).

The following primary antibodies were used: anti-albumin rabbit polyclonal antibody (A0001; DakoCytomation Denmark A/S, Glostrup, Denmark); anti-prosurfactant protein C (proSP-C: marker for type 2 alveolar epithelial cells) rabbit polyclonal antibody (AB3786; Chemicon International, Millipore); anti-α-fetoprotein (AFP) mouse monoclonal antibody (AB3786; Vector Laboratories); and anti-β-catenin mouse monoclonal antibody (610153; BD Transduction Laboratories, BD Biosciences).

MDM2 was stained using an anti-MDM2 mouse monoclonal antibody (sc-965; Santa Cruz Biotechnology), an M.O.M immunodetection kit (Vector Laboratories) and a DAB peroxidase substrate solution (Vector Laboratories). Anti-FEATΔN and anti-FEATΔC antibodies are those recognizing different sites on the FEAT protein, and their epitopes do not overlap with each other. As a result of staining with these antibodies, the same areas were stained in the mouse brain and testis, suggesting the specificity of FEAT staining.

### Preparation of genomic DNA for array-CGH

As described in Tomioka et al., 2008, genomic DNA was prepared from mouse tissues. Namely, in 2 ml microtubes, the minced tissues were suspended in 1 ml of ice-cold TEN buffer (50 mM Tris-HCl (pH 8.0), 1 mM EDTA, pH 8.0, 100 mM NaCl) and shaken gently with 50 µl of 10% SDS. 25 µl of 20 mg/ml proteinase K was added and the tissues were incubated overnight at 50°C. Then, 1 ml of TE saturated phenol (NIPPON GENE Co., Ltd., Tokyo, Japan) was added, and each sample was rotated at room temperature for 1 hour and then centrifuged at 8000 rpm at 15°C for 15 minutes to collect the aqueous layer. After repeating the above phenol extraction, the aqueous layer was collected and mixed with 1 ml of chloroform isoamyl alcohol. The mixture was rotated at room temperature for 1 hour and centrifuged at 3000 rpm at 15°C for 10 minutes to collect the aqueous layer. 2 ml of ethanol which had been cooled to -20° was added and shaken gently to precipitate genomic DNA. This precipitate was transferred with a Pasteur pipette to a 1.5 ml microcentrifuge tube containing 1 ml of 70% ethanol and centrifuged at 15000 rpm at 4°C for 5 minutes. The pellet was suspended again in 200 µl of TE buffer (10 mM Tris-HCl (pH 8.0), 1 mM EDTA) and dissolved by being rotated overnight at room temperature. The absorbance at 260 nm was measured with a DU 800 spectrophotometer (Beckman Coulter, Inc., Fullerton, California) to determine the DNA concentration. The purity of DNA was evaluated by electrophoresis of 200 ng DNA on a 0.8% agarose gel containing 0.5 µg/ml ethidium bromide. Only DNA samples without smearing were used for the subsequent steps.

### Array-CGH

DNA labeling and hybridization were performed on 244k slide format 60-mer oligonucleotide microarrays for array-CGH (Agilent Mouse Genome CGH Microarray Kit 244A) using the following DNAs: mouse hepatocellular carcinoma-derived genomic DNA (experimental sample) and normal C57BL/6 mouse liver-derived genomic DNA (control sample). In accordance with the instructions of Agilent Technologies (Santa Clara, California), the microarrays were scanned with a microarray scanner and data were extracted using Feature Extraction software and analyzed using CGH analytics 3.4 software.

### Tissue arrays

Tissue arrays on which formalin-fixed and paraffin-embedded various normal and tumor tissue fragments were spotted (Blow, 2007) were purchased from US Biomax, Inc. (Ijamsville, Maryland). The following microarrays were used: MC5001 (high-density multiple organ cancer and normal tissue microarray); BR721 (breast cancer tissue microarray with self-matching cancer adjacent normal tissues and normal tissue controls); BC05021 (colon adenocarcinoma (combination of malignant and normal samples); and LV803 (liver cancer (combination of cancer tissue, cancer adjacent tissue and normal tissue).

### Analysis of p53 and β-catenin mutations in mouse hepatocellular carcinoma

Using an RNeasy Mini kit (Qiagen), total RNA was isolated from mouse hepatocellular carcinoma tissues immediately after being excised. PCR was performed with KOD-Plus-DNA polymerase. For amplification of p53 ORF, the following two primer sets were used:
p53 forward-1: 5'-CTAGCATTCAGGCCCTCATC-3' (SEQ ID NO: 16)
p53 reverse-1: 5'-GAAAAGTCTGCCTGTCTTCCA-3' (SEQ ID NO: 17)
p53 forward-2: 5'-GCTCCTCCCCAGCATCTTAT-3' (SEQ ID NO: 18)
p53 reverse-2: 5'-TTGTGTCTCAGCCCTGAAGT-3' (SEQ ID NO: 19)

The amplification products were subjected to direct sequencing. To confirm gene mutations, the mouse p53 gene was further sequenced using genomic DNA and the following primer set:
Forward: 5'-GCTCCGATGGTGATGGTAAG-3' (SEQ ID NO: 20)
Reverse: 5'-TAGCACTCAGGAGGGTGAGG-3' (SEQ ID NO: 21)

KOD-Plus-DNA polymerase, hepatocellular carcinoma genomic DNA prepared for array-CGH and the following primer set were used in PCR to amplify the region covering exons 1 to 3 of the mouse β-catenin gene. Exon 2 included in this region is a hot spot where mutations frequently occur (de La Coste, A. et al. (1998) Proc. Natl. Acad. Sci. U.S.A. 95, 8847-8851).
Forward: 5'-CCTGAAGCTCAGCGCACA-3' (SEQ ID NO: 22)
Reverse: 5'-CCCTCATCTAGCGTCTCAGG-3' (SEQ ID NO: 23)

Nested PCR was further performed using KOD-Plus-DNA polymerase and the following primers:
Forward: 5'-TCTCCTTGGCTGCCTTTCTA-3' (SEQ ID NO: 24)
Forward: 5'-GTCACACAGCCCTGTCAAGA-3' (SEQ ID NO: 25)

The resulting amplification products were subjected to direct sequencing.

### Statistical analysis

Statistical analysis was performed using a Microsoft Excel add-in package, Statcel2 (OMS-publishing, Tokorozawa, Japan).

### <2. Experimental results>

### Biochemical purification of FEAT protein

Analysis on regulatory molecules of nuclear apoptosis in a cell-free system indicated that apoptosis-promoting activity was attenuated in the middle of active fractions, along with appearance of two peptides whose apparent molecular weight was 74 kDa and 66 kDa, respectively (Figure 1A). When these peptides were purified, the 66 kDa protein was found to be a molecule called SWAP-70 responsible for regulating apoptosis in B cells. The 74 kDa protein was found to be a rat homolog of the protein called "comparative gene identification-01 (CGI-01)" (Lai, C.H. et al., (2000) Genome Res. 10, 703-713), "KIAA0859" or "methyltransferase like 13 (METTL13)." Based on its expression pattern and functions, this protein was designated as "FEAT" (faint expression in normal tissues, aberrant overexpression in tumors).

FEAT contains two S-adenosylmethionine-binding motifs (SAM-binding motifs). The SAM-binding motif is a motif characteristic of methyltransferase and related enzymes thereof (Kagan, R.M., and Clarke, S. (1994) Arch. Biochem. Biophys. 310, 417-427). The structure of FEAT is well conserved across species (Figure 1B). The bars I, postI, II and III indicated above the alignments shown in Figure 1B represent SAM-binding motifs. In addition, the arrows indicated above the alignments represent the cleavage sites for caspase-3. The alignments were prepared using ClustalW and Boxshade software.

In either the full-length or truncated form of the FEAT protein, protein methyltransferase activity or spermidine/spermine synthase activity was not observed (data not shown). The complementation test in yeast suggested that FEAT was not ubiquinone synthetase (Figure 1C).

### Caspase-3-mediated cleavage of FEAT

Upon induction of apoptosis in COS-7 cells with staurosporine (STS), FEAT expressed by transfection was cleaved (Figure 2A). FEAT transcribed/translated *in vitro* was cleaved by caspase-3, but not by caspase-6 (Figure 2B). In MCF-7 cells deficient in caspase-3, FEAT was minimally cleaved, but co-expression of procaspase-3 allowed efficient cleavage of FEAT (Figure 2C). Site-directed mutagenesis experiments were conducted to identify caspase cleavage sites in the human FEAT protein (Figure 2D). In Jurkat T cells undergoing Fas- and STS-induced apoptosis, their endogenous FEAT was cleaved (Figure 2E). These results indicated that FEAT was cleaved by caspase-3 during apoptotic cell death (Figure 2F).

### FEAT-induced suppression of apoptotic cell death

The human FEAT gene is located at chromosome 1q24.3, but this site is frequently amplified in malignant lymphoma and associated with poor prognosis. In lymphoma cell lines, the expression level of FEAT mRNA was correlated with suppression of STS-induced apoptosis (Figure 3A). It is known that caspase-mediated cleavage of anti-apoptotic kinases and phosphatases induces apoptosis through blockage of survival signals and generation of apoptosis-inducible peptide fragments (Kurokawa, M., and Kornbluth, S. (2009) Cell 138, 838-854). To confirm whether or not FEAT or caspase-3-cleaved fragments thereof would affect apoptosis, cDNA encoding FEAT with or without a mutation(s) in the caspase-3 cleavage site(s) was introduced into Karpas 299 cells. Karpas 299 cells expressing the D274A/D288A mutant showed a significant reduction in STS-induced apoptosis (Figure 3B) (Mean ± SEM (*, P < 0.01; **, P < 0.025, n = 3; paired t-test)). Upon introduction of a D112A mutation into the D274A/D288A mutant, the D274A/D288A mutant lost its ability to suppress apoptosis (D112A/D274A/D288A). This result suggested that D112 was essential for anti-apoptotic functions.

Karpas 299 cells were found to endogenously express FEAT, as analyzed by Western blotting (data not shown). When a search was made for cells not expressing FEAT, almost all cancer-derived cell lines were found to express FEAT (Figure 4A), and hence neutrophils were used for studies (Figure 4B: peripheral blood monocytes taken from a patient with acute lymphocytic leukemia (ALL) and neutrophils taken from normal volunteers (normal-1 to normal-4)). Protein introduction of wild-type FEAT (FEAT wt) and FEATΔN (amino acids (aa) 289-699: corresponding to a fragment generated by caspase-3 cleavage) significantly suppressed spontaneous apoptosis in neutrophils (Figure 4C) (means ± SEM; *, P < 0.002, n = 9; **, P < 0.0002, n = 9; ***, P < 0.02, n = 4; ****, P < 0.02, n = 4; paired t-test). The D274A/D288A mutant was found to have a more potent suppressive effect than FEAT wt or FEATΔN. In contrast, FEATΔC (aa 1-274) showed no suppressive effect against apoptosis, but was found to interfere with the anti-apoptotic effect of FEATΔN (FEATΔN+C in Figure 4C). Knockdown of FEAT expression by short hairpin RNAs (Figure 3C) enhanced STS-induced apoptosis in HeLa cells (Figure 3D) (Mean ± SEM (*, P < 0.01; **, P < 0.025, n = 3; paired t-test)). This result indicated that endogenous FEAT also suppressed apoptotic cell death. In contrast, knockdown of FEAT did not affect cell cycle and cell growth. In view of the foregoing, it is concluded that FEAT has the ability to suppress apoptosis and this suppression ability is lost upon caspase cleavage at D274 and D228 of FEAT.

Figure 3A shows the results of RT-PCR on lymphoma cell lines, which indicate that the expression level of FEAT is inversely correlated with the incidence of apoptosis.

FEAT was found to be localized diffusely in the cytoplasm and nuclei and partially in ERs, as analyzed by immunostaining (Figure 3E). This result was consistent with the prediction obtained from PSORT II analysis to the effect that FEAT was localized in ERs with a probability of 34.8%. FEAT was not co-localized with actin and mitochondria (Figure 3E: magnification × 400).

### Abnormal expression of FEAT in human cancer cells

FEAT was found to correspond to a TGACCTCCAG tag (Velculescu, V.E. et al. (1999) Nat. Genet. 23, 387-388), which is used in SAGE transcriptome experiments for serial analysis of human gene expression. The TGACCTCCAG tag shows a higher expression level in human colorectal cancer, brain tumor, breast cancer, lung cancer and melanoma than in normal tissues. When human normal cells were analyzed by Western blotting, weak expression of FEAT was observed only in the testis, brain and liver (Figure 5A). This result obtained by Western blotting analysis is consistent with the result of messenger RNA expression (Lai, C.H. et al., (2000) Genome Res. 10, 703-713). In contrast to normal cells, most types of human cancer tissues showed moderate to high expression of the FEAT protein (Figure 5B), indicating that FEAT is a ubiquitous protein involved in tumor development.

To examine whether FEAT has any function in tumorigenesis, NIH3T3 cells in which the FEAT protein is expressed at low level was engineered to overexpress FEAT, and microarrays were used to comprehensively analyze alterations in transcriptional profiles. Figure 5C shows the results of Western blotting with anti-FEAT antibody performed on NIH3T3 cells transfected with control plasmids (ΔccdB-1, ΔccdB-2 and ΔccdB-3) as well as human FEAT cDNA expression plasmids (FEAT-3, FEAT-15 and FEAT-20). Gene set enrichment analysis (GSEA) (Subramanian, A. et al. (2005) Proc. Natl. Acad. Sci. U.S.A. 102, 15545-15550) indicated that FEAT overexpression enhanced the signals of the receptor tyrosine kinase (RTK) and Hedgehog signaling pathways (Figure 5D (left panel: enrichment plots; right panel: heat maps) and Table 1). These signaling pathways are known to play important roles in the development and maintenance of tumors (Vogelstein, B., and Kinzler, K.W. (2004) Nat. Med. 10, 789-799; Rubin, L.L., and de Sauvage, F.J. (2006) Nat. Rev. Drug Discov. 5, 1026-1033). These results suggest that FEAT has a potential as a tumor-promoting protein.

**Table 1**

| Gene expression signatures enriched in FEAT-overexpressed or control NIH3T3 cells | | |
|---|---|---|
| Gene sets database: GO biological process | ES | FDR *q*-value |
| ***Enriched in FEAT-averexpressed NIH3T3 cells*** | | |
| TRANSMEMBRANE_RECEPTOR_PROTEIN _TYROSINE_KINASE_SIGNALING_PATHWAY | 0.55 | 0.079 |
| RESPONSE_TO_EXTERNAL_STIMULUS | 0.38 | 0.163 |
| RESPONSE_TO_WOUNDING | 0.41 | 0.170 |
| INFLAMMATORY_RESPONSE | 0.47 | 0.170 |
| | | |

| ***Enriched in control NIH3T3 cells*** | | |
|---|---|---|
| LIPID_BIOSYNTHETIC_PROCESS | 0.44 | 0.117 |
| COENZYME_METABOLIC_PROCESS | 0.47 | 0.233 |

| Gene sets database: KEGG | ES | FDR *q*-value |
|---|---|---|
| ***Enriched in FEAT-overespressed NIH3T3 cells*** | | |
| N_GLYCAN_BIOSYNTHESIS | 0.51 | 0.127 |
| HEDGEHOG_SIGNALING_PATHWAY | 0.54 | 0.216 |
| | | |

| ***Enriched in control NIH3T3 cells*** | | |
|---|---|---|
| BIOSYNTHESIS_OF_STEROIDS | 0.72 | 0.002 |
| PROTEASOME | 0.57 | 0.094 |
| GLYCOSYLPHOSPHATIDYLINOSITOL_ANCHOR _BIOSYNTHESIS | 0.51 | 0.130 |
| PPAR_SIGNALING_PATHWAY | 0.45 | 0.135 |
| PYRUVATE_METABOLISM | 0.50 | 0.142 |
| NEUROACTIVE_LIGAND_RECEPTOR_INTERACTION | 0.49 | 0.146 |
| PROPANOATE_METABOLISM | 0.50 | 0.170 |
| FATTY_ACID_METABOLISM | 0.43 | 0.171 |
| CITRATE_CYCLE | 0.46 | 0.173 |
| GLYCAN_STRUCTURES_BIOSYNTHESIS_2 | 0.44 | 0.189 |
| VALINE_LEUCINE_AND_ISOLEUCINE_DEGRADATION | 0.40 | 0.210 |
| AMINOSUGARS_METABOLISM | 0.45 | 0.226 |

| | | |
|---|---|---|
| ES, enrichment score; FDR, false discovery rate. | | |

Figure 5D shows the results of GSEA analysis for genome-wide expression profiling of NIH3T3 cells transfected with human FEAT cDNA. The left and right panels of Figure 5D show enrichment plots and heat maps, respectively, which list gene sets with an enrichment score (ES) >0.5 and a false discovery rate (FDR) q-value <0.25.

### FEAT upregulation-induced promotion of tumorigenesis in vivo

To evaluate whether the enhanced expression level of FEAT contributes to tumorigenesis, transgenic (Tg) mice expressing human FEAT were prepared (Hanahan, D. et al (2007) Genes Dev. 21, 2258-2270). Human FEAT was expressed under the control of a promoter which is active in various human tissues. The Tg mice were born at the ratio expected according to the Mendel's laws of heredity. Except for the fact that congenital cataract was observed in about 15% of newborn Tg mice, all other organs developed normally without any histological abnormalities until 9 months of age. Among six primary Tg mice, one female (line 10) and one male (line 14) were determined to be sterile as a result of crossing with a plurality of normal C57BL/6 mice. The first-generation progeny of one primary Tg male (line 23) and the second-generation progeny of one primary Tg male (line 18) were found to be sterile.

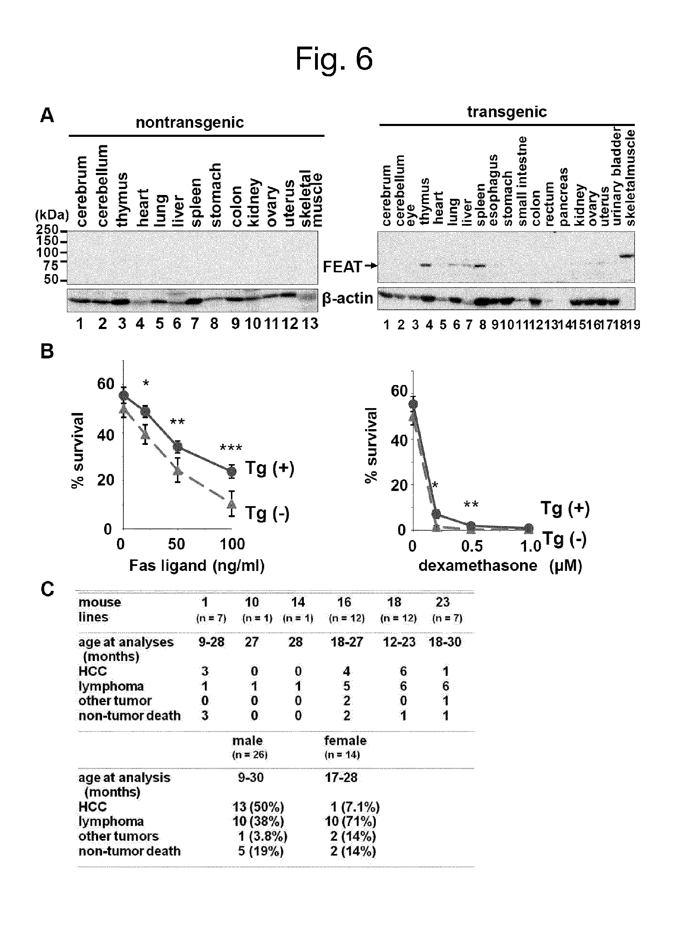

Sperm of the second-generation Tg mice (lines 1, 16 and 18) were observed under a microscope, indicating that the motility of their sperm was reduced in comparison with their littermate non-transgenic (non-Tg) mice. In Western blotting analysis, the Tg mice showed human FEAT expression in their thymuses, spleens, livers and lungs (Figure 6A: left panel, non-Tg mice; right panel, Tg mice). Thymocytes derived from the Tg mice showed significant decreases in Fas ligand-induced cell death and glucocorticoid-induced cell death (Figure 6B) (Fas ligand (left panel): means ± SEM, *, P < 0.025; **, P < 0.05; ***, P < 0.025, n = 5; paired t-test; dexamethasone (right panel): means ± SEM, *, P < 0.04; **, P < 0.05, n = 6; paired t-test)), indicating that FEAT expression in the Tg mice suppressed apoptosis. Tumors developed in the primary, first-generation and second-generation FEAT Tg mice are shown in Figure 6C (HCC: hepatocellular carcinoma).

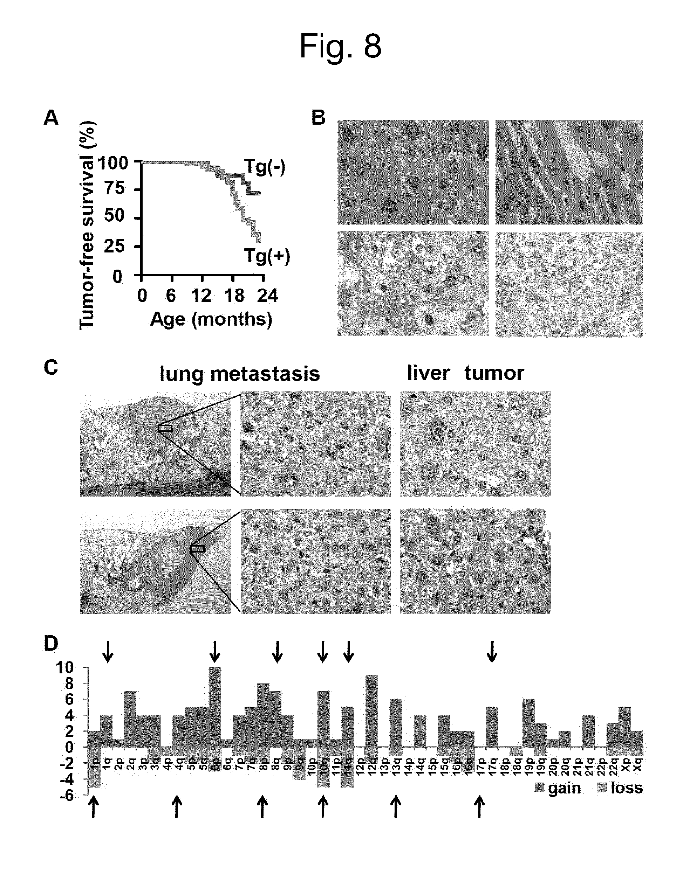

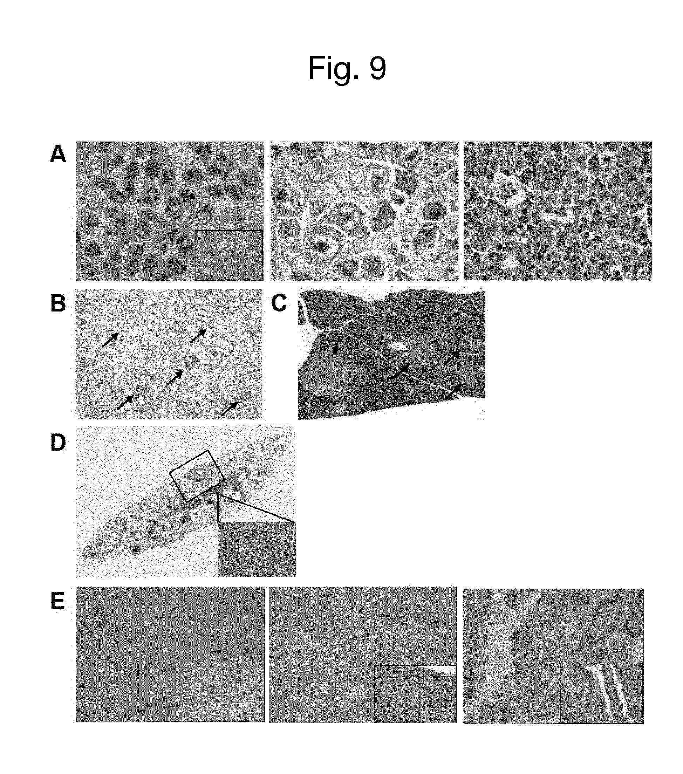

Upon reaching 12 months of age, the Tg mice began to develop hepatocellular carcinoma (HCC) in their livers (Figure 7A: arrows) and malignant lymphoma (Figure 7C: arrows). In contrast, in their littermate non-Tg mice, the onset of hepatocellular carcinoma was not observed, while lymphoma was observed only at very low incidence (18%, 3 of 17 mice). Thus, the Tg mice were more likely to develop tumors or had shorter life spans than the littermate non-Tg mice (Figure 8A: Kaplan-Meier plots for tumor-free survival of Tg mice (red line: n = 40) and littermate non-Tg mice (blue line: n = 17) (P < 0.04, log-rank test)). Hepatocellular carcinoma and lymphoma in Tg mice were observed in the progeny (line) of four distinct primary Tg mice (Figure 6C). This denies the possibility that tumors were formed as a result of activating endogenous cancer genes upon transgene insertion. Human hepatocellular carcinoma more often develops in males (Naugler, W.E. et al., (2007) Science 317, 121-124), and the same tendency was also observed in mice (Figure 6C). As in the case of human hepatocellular carcinoma, most cases of mouse hepatocellular carcinoma were found to be well-differentiated tumors (upper panels of Figure 8B). Moreover, in mouse hepatocellular carcinoma, variants were also observed, such as those of clear cell type (lower left panel of Figure 8B) and those with cytoplasm inclusion bodies (lower right panel of Figure 8B). Lung metastasis was also observed in two cases of mouse hepatocellular carcinoma (Figure 8C). Most cases of lymphoma were found to belong to centroblastic or immunoblastic diffuse B-cell lymphoma and Burkitt-like B-cell lymphoma with a so-called "starry-sky appearance" (Figure 9A). These variants both expressed CD45R (B220) (100%, 15 of 15 mice) (inset of Figure 9A) and showed clonal rearrangement of the immunoglobulin genes (83%, 5 of 6 mice). These features are identical with the features of B-cell lymphoma most often seen in human lymphoma patients. None of lymphomas used in the experiment was positive for CD3 (0%, 0 of 7 mice). CD45R- and CD3-negative variants with giant cells were also observed (Figure 9B). These cases of lymphoma were highly invasive and frequently infiltrated into the pancreas (Figure 9C). Two mice had both lymphoma and hepatocellular carcinoma (Figure 9D), and one of these mice also had lung adenocarcinoma (Figure 9E). The FEAT transgene whose expression was driven by hepatocellular carcinoma was sequenced, indicating that there was no mutation (0%, 0 of 5 mice). This suggests that structural changes in FEAT are not required for tumorigenesis. Unlike CF-1 and C3H inbred mice, C57BL/6 inbred mice are known to rarely develop hepatocellular carcinoma (Anisimov, V.N. et al., (2005) Nat. Rev. Cancer 5, 807-819). Thus, the FEAT Tg mice are a unique mouse model which allows the onset of both hepatocellular carcinoma and lymphoma with high penetrance in C57BL/6 inbred mice.

Figures 7A and 7C show the gross appearance of the primary, first-generation and second-generation FEAT Tg mice with hepatocellular carcinoma and malignant lymphoma, respectively. The arrows in Figure 7A represent tumors developed in the liver, while the arrows in Figure 7C represent mesenteric, mediastinal or axillary lymph nodes as well as livers and spleens, which were enlarged due to malignant lymphoma. The photograph shown in Figure 7B is a photograph showing a male Tg mouse at 9 months of age which was found by chance to have early hepatocellular carcinoma when dissected for sperm storage (lower left panel of Figure 8B). This mouse has a small tumor (arrow) in the liver. This indicated that liver cancer would develop earlier than 12 months of age. Figure 7D shows photomicrographs of lymphoma (upper panel) and rhabdomyosarcoma (lower panel) developed simultaneously in the same Tg mouse (H&E staining: × 400 magnification). Figure 7E shows Kaplan-Meier plots for tumor-free survival of Tg mice with hepatocellular carcinoma (red line) and Tg mice with lymphoma (blue line).

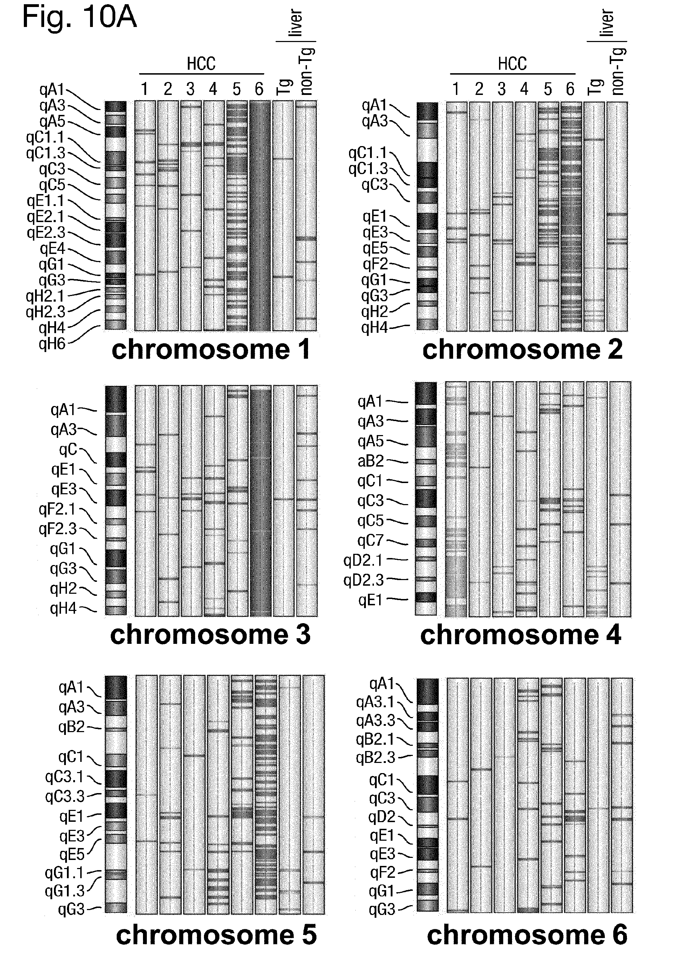

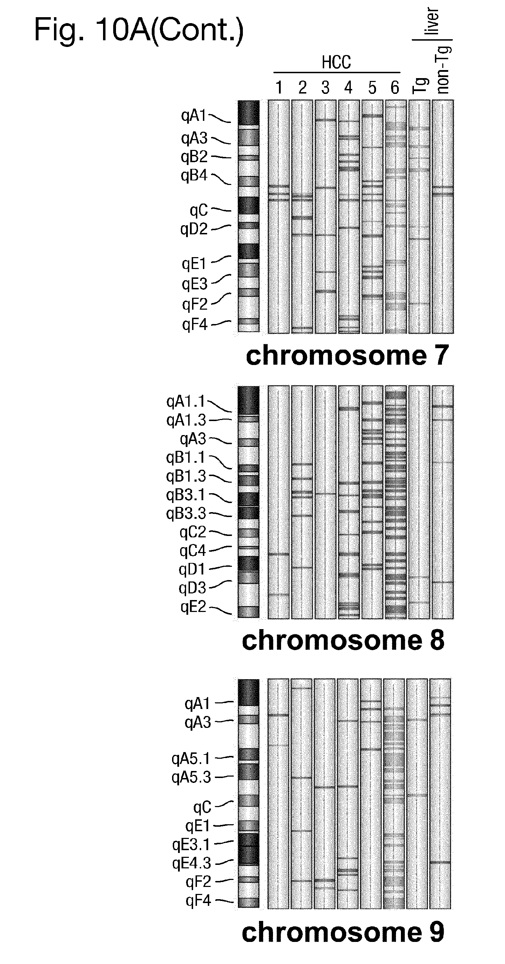

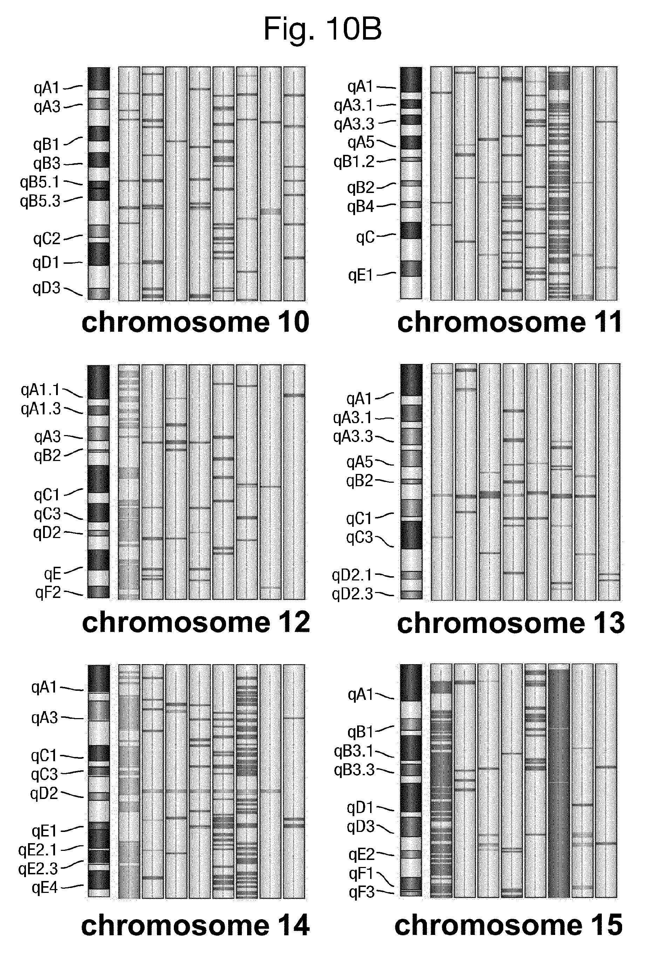

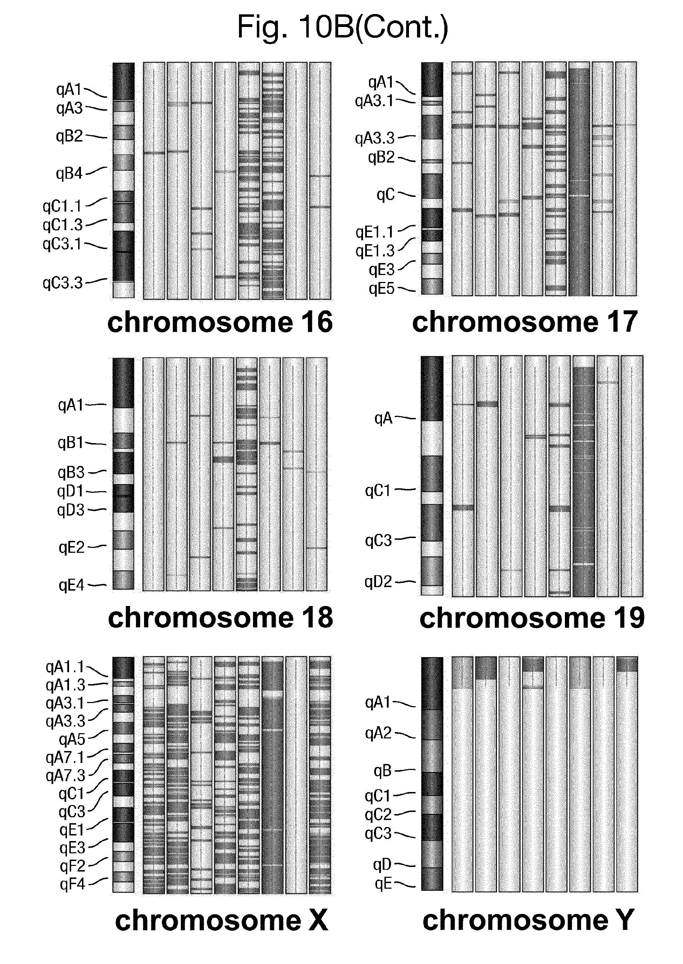

Figure 10 is graphical aberration showing amplifications (red) and deletions (green) in mouse chromosomes. The graphical aberration shows the results analyzed for DNAs derived from hepatocellular carcinoma (HCC) in six FEAT Tg mice, normal liver of one Tg mouse and normal liver of one non-Tg mouse by CGH Analytics 3.4 software (z-score threshold: 2.5) (Agilent Technologies).

### FEAT Tg mice as a model for human hepatocellular carcinoma

With the use of model mice developing cancers whose physiological and molecular features closely resemble those of human cancer patients, it is possible to determine the causes of human cancers, to develop methods for cancer prevention, and to develop and test new therapies. For this purpose, studies were conducted to evaluate whether or not hepatocellular carcinoma in FEAT Tg mice has genetic changes similar to those of human hepatocellular carcinoma.

To examine whether or not mouse hepatocellular carcinoma has chromosomal changes similar to those of human patients, amplifications (gain) and deletions (loss) (copy number alterations (CNAs)) in genomic regions were analyzed by microarray-based comparative genomic hybridization (array comparative genomic hybridization (array-CGH)). Macroscopically, mouse hepatocellular carcinoma had more amplifications than deletions due to genomic instability (Figure 10). This result was similar to that of hepatocellular carcinoma in humans (Zender, L. et al. (2006) Cell 125, 1253-1267). In contrast, the livers from Tg mice without hepatocellular carcinoma showed no significant copy number alteration. The foregoing results indicated that genomic instability occurred through the process of hepatocarcinogenesis rather than due to direct effects of the FEAT transgene. Recent studies have reported that tandem duplications of 3 kb to 1 Mb small regions are observed in common as structural changes in human breast cancer genomes (Stephens, P.J. et al. (2009) Nature 462, 1005-1010). In this high-resolution array-CGH, the amplification and deletion of small chromosomal regions were also observed in mouse hepatocellular carcinoma. Copy number alterations in these small regions involved the following 18 types of cancer-related genes (http://www.sanger.ac.uk/genetics/CGP/Census/): MDS1, PDGFRA, PIK3R1, JAZF1, WHSC1L1, HOOK3, PCM1, MLLT3, PTEN, CBL, ERCC5, ERCC4, CYLD, CBFB, BRIP1, MLLT1, TMPRSS2 and NF2 (Table 2). TTN, SKP2, EED1 and PVT1, which are not registered as cancer-related genes, would also contribute to tumorigenesis.

**Table 2**

| Genes showing copy number alterations in hepatocellular carcinoma of FEAT Tg mice | | |
|---|---|---|
| Syntenic human chromosome | gain | loss |
| 1p | CDC42, EPHA8 | ZNF697, SGIP1, GPR157, CAMTA1, RNF207 |
| 1q | CHIT1, ARID4B, PLD5, SDCCAG8 | |
| 2p | ABCG8 | |
| 2q | TTN, ALSIN, TMEM169, FN1, KCNE4, IRS1, ASB1 | |
| 3p | ROBO2, SFMBHT1, THRB, ZFYVE20 | |
| 3q | FNDC3B, MDS1 SPATA16, TNFSF10 | PLOD2, GFM1 |
| 4p | | SLIT2 |
| 4q | PDGFRA, IGFBP7, ENOPH1, SMAD1 | TBCKL |
| 5p | NIPBL, SKP2, IL7R, KPL2, CDH18 | HCN1, PRKAA1 |
| 5q | PPAP2A, PIK3R1, SCAMP1, SLCO4C1, ADAMTS2 | GZMK, CKMTS |
| 6p | TREML4, LRFN2, HCRTR2, C6orf138, CUL7, Unc5cl, RUNX2, SRF, PTK7, DDX16 | KCNK5, LRFN2, NMOR2 |
| 6q | SHPRH | |
| 7p | PKDIL1, AEBP1, NPCIL1, ADCY1 | JAZF1 |
| 7q | KRIT1, PNPLA8, ANKRD7, KCP, KLRG2 | ADCK2, SMARCD3 |
| 8p | WHSC1L1, ERLIN2, SGK196, HOOK3, UBXN8, DPYSL2, DCTN6, PCMI, CLDN23 | FBXO25 |
| 8q | PRKDC, NIPAL2, DORFIN, EDD1, PVT1, MTSS1, NFKBIL2 | |
| 9p | FRMPD1, MLLT3, ZDHHC21, KIAA2026 | MTAP, RRAGA |
| 9q | RAB14 | Cl1orf53, PTPRD,LRSAM1, SLC34A3 |
| 10p | ANKRD26 | |
| 10q | TSPAN14, HPS1, PTEN, AFAP1L2, TACC2, STK32C,INPP5A | UNC5B, LOC219347, DUXA, XPNPEP1, SEC23IP |
| 11p | LRRC4C | MRPS33 |
| 11q | CAPN5, DYNC2H1, ME3, GRIA4, MMP12 | OTUB1, CCDC67, ELMOD1, TRIM29, CBL |
| 12q | DAZAP2, MAP3K12, HOXC4, CS, HEM1, C12orf26, SCYL2, UHRF1BP1L, CCDC53 | LYZ, ROBO3 |
| 13q | CENPJ, MIPEP, DZIP1, ERCC5, EFNB2, MYO16 | LRRC63 |
| 14q | SGPP1, FAM161B, GSTZ1, FLRT2 | |
| 15q | TUBGCP5, MYO9A, MAP2K5, STRA6 | SGK269 |
| 16p | MAPK3, ERCC4 | GSG1L, ZP2 |
| 16q | ITFG1, CYLD | NETO2, CDH5, CBFB |
| 17q | BRIP1, CSH1, TMEM104, C17orf28, ATP5H | |
| 18q | | CCBE1 |
| 19p | TMEM161A, C19orf57, PODNL1, DAND5, MRI1, MLLT1 | |
| 19q | VSTM2B, KLK14, AP2A1 | PLAUR |
| 20p | C20orf94 | |
| 20q | TMEM189, CABLES2 | |
| 21q | DONSON, BACE2, MX2, TMPRSS2 | |
| 22q | PITPNB, NF2, TRABD | |
| Xp | CNKSR2, RRAGB, SHROOM4, SLC9A7, OA1 | CXorf23 |
| Xq | HEPH, TAF1 | ZC3H12B |

For direct comparison of copy number alterations in small regions between mouse hepatocellular carcinoma and human hepatocellular carcinoma, a list was made of genes contained in the syntenic regions of human chromosomes corresponding to the regions with copy number alterations common to mouse hepatocellular carcinoma cases (Table 2). Figure 8D shows the number of genes present in the syntenic regions of human chromosomes corresponding to the copy number alterations detected by array-CGH on hepatocellular carcinoma in FEAT Tg mice (n = 6). The syntenic regions shown in Figure 8D cover almost all of the mutated chromosomal regions which have been suggested to be involved in human hepatocellular carcinoma (Farazi, P.A., and DePinho, R.A. (2006) Nat. Rev. Cancer 6, 674-687). Moreover, the results of array-CGH on mouse hepatocellular carcinoma were very similar to the previously reported results of array-CGH on human hepatocellular carcinoma (Zender, L. et al. (2006) Cell 125, 1253-1267). In view of the fact that copy number alterations were confirmed in the gene regions common across species, hepatocellular carcinoma in these Tg mice faithfully mimics hepatocellular carcinoma in human patients, thus suggesting that the FEAT Tg mice are an useful animal model for human hepatocellular carcinoma.

### Enhanced FEAT expression in various human cancer tissues

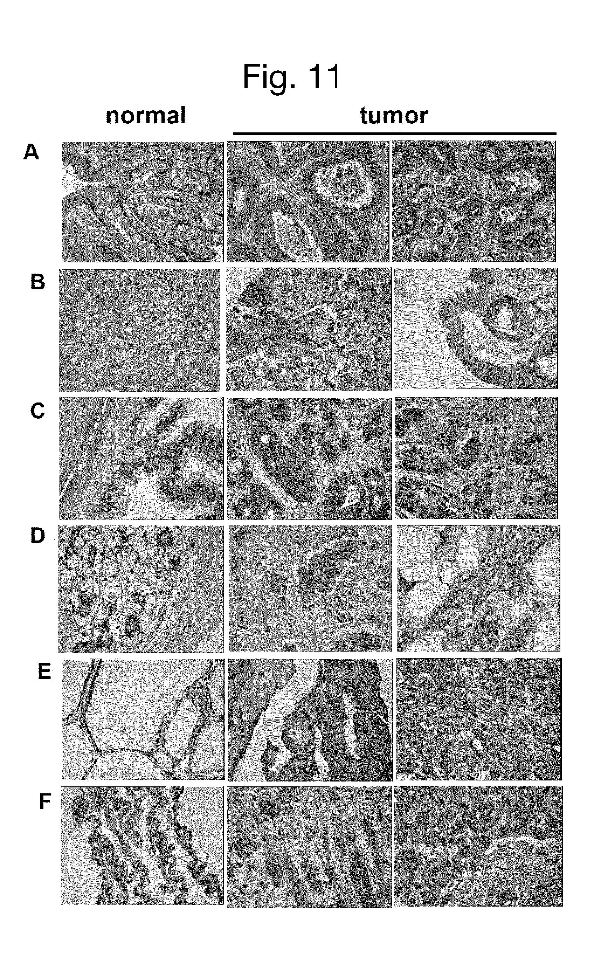

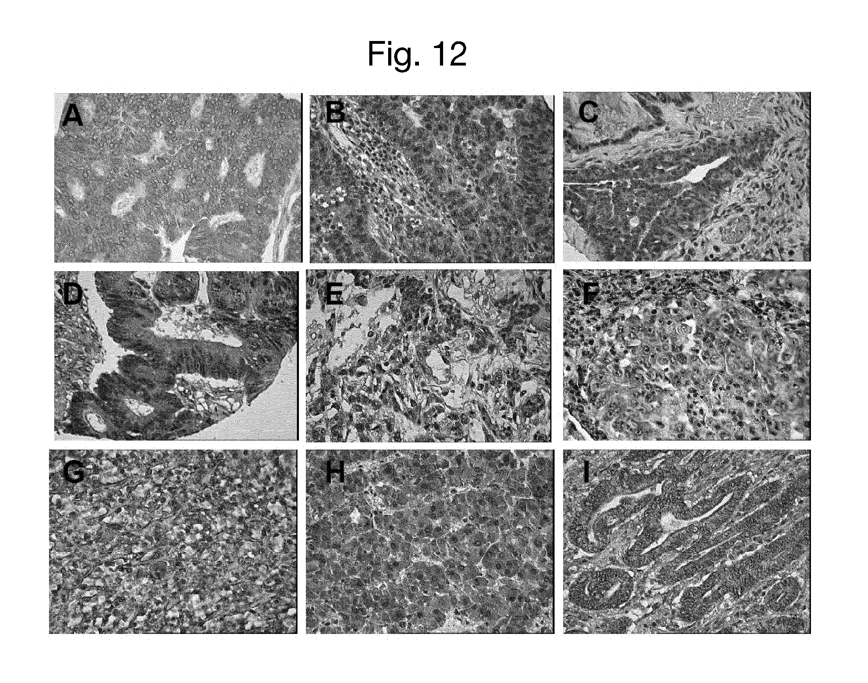

To estimate the range of human cancers in which FEAT contributes to their onset, FEAT expression levels in human normal tissues, various cancers and their subtypes were examined using tissue arrays. FEAT expression was found to be significantly enhanced in colorectal cancer, pancreatic cancer, prostate cancer, breast cancer, ovarian cancer, thyroid cancer and non-small cell lung cancer, in comparison with normal tissues (Figure 11, Figure 12 and Table 3). FEAT expression was enhanced in cancer cells, but not in non-neoplastic stromal cells or normal cells adjacent to cancer tissues. Thus, enhanced FEAT expression is a feature common to a wide range of human cancers, suggesting that this feature is an imperative process in the development of most cancers. Surprisingly, in human hepatocellular carcinoma, FEAT overexpression occurs prior to tumor-induced phenotypic changes; and hepatocytes adjacent to hepatocellular carcinoma in liver cirrhosis tissues actually showed high level expression of FEAT (Figure 13). In Figure 13, the numerals in the figure represent the age of patients, while M represents the sex (male) of the patients. This result would reflect the progress of carcinogenesis in these liver cirrhosis tissues. Moreover, significant FEAT overexpression was also observed *in situ* in breast intraductal carcinoma (early progressive invasion) (Figure 12A). The foregoing results suggest that FEAT is involved in diverse human cancers from the precancerous stage or an early stage of tumorigenesis.

**Table 3**

| FEAT expression in human normal and tumor tissues | | | | | | |
|---|---|---|---|---|---|---|
| tissue | | - | ± | + | 2+ | statistics |
| colon | adenocarcinoma | 7 | 13 | 15 | 2 | *P* < 0.03 (Mann-Whitney's U test; MWU) |
| | normal | 3 | 7 | 0 | 0 | |
| pancreas | adenocarcinoma | 0 | 7 | 8 | 3 | *P* < 0.01 (MWU) |
| | normal | 1 | 5 | 0 | 0 | |
| prostate | adenocarcinoma | 4 | 7 | 6 | 1 | *P* < 0.01 (MWU) |
| | normal | 6 | 1 | 0 | 0 | |
| breast | invasive ductal carcinoma | 3 | 7 | 10 | 3 | *P* < 0.02 (Kruskal-Wallis test; KW) |
| | infiltrating lobular carcinoma | 2 | 5 | 3 | 1 | |
| | intraductal carcinoma | 0 | 0 | 0 | 1 | |
| | normal | 7 | 7 | 3 | 0 | |
| ovary | serous papillary | | | | | |
| | cystadenocarcinoma mucinous papillary | 4 | 9 | 1 | 0 | *P* < 0.04 (KW) |
| | cystadenocarcinoma | 0 | 1 | 2 | 0 | |
| | endometrioid carcinoma | 0 | 0 | 1 | 0 | |
| | endodermal sinus carcinoma | 1 | 2 | 1 | 0 | |
| | ovarian teratoma | 0 | 2 | 1 | 0 | |
| | normal | 4 | 1 | 0 | 0 | |
| thyroid | papillary carcinoma | 2 | 1 | 4 | 3 | *P* < 0.02 (KW) |
| | follicular carcinoma | 2 | 5 | 2 | 0 | |
| | normal | 5 | 1 | 0 | 0 | |
| lung | squamous cell carcinoma | 0 | 2 | 5 | 0 | *P* < 0.01 (KW) |
| | adenocarcinoma | 0 | 5 | 3 | 2 | |
| | large cell carcinoma | 0 | 1 | 4 | 0 | |
| | normal | 4 | 1 | 0 | 0 | |
| kidney | clear cell carcinoma | 0 | 6 | 3 | 1 | *P* = 0.473 (KW) |
| | granular cell carcinoma | 0 | 5 | 3 | 2 | |
| | normal | 0 | 4 | 1 | 0 | |

| | | | | | | |
|---|---|---|---|---|---|---|
| -, negative; ±, weakly positive; +, positive; 2+, strongly positive | | | | | | |

### INDUSTRIAL APPLICABILITY

FEAT is not expressed in normal cells and its expression is limited to tumor cells or precancerous cells. In addition, FEAT is expressed from an early stage of tumorigenesis. Thus, the test reagent of the present invention allows tumor detection at an early stage through detection of FEAT-expressing cells.

Moreover, the pharmaceutical composition of the present invention allows not only prevention of further development of tumors, but also treatment of tumors which have already developed, through killing of FEAT-expressing cells.

Further, the FEAT-expressing tumor animal model of the present invention is an animal model that faithfully mimics pathological features of human hepatocellular carcinoma or malignant lymphoma, and is useful for studies of these cancer and tumor.

### Sequence Listing Free Text

SEQ ID NO: 9: synthetic DNA
SEQ ID NO: 10: synthetic DNA
SEQ ID NO: 11: synthetic DNA
SEQ ID NO: 12: synthetic DNA
SEQ ID NO: 13: synthetic DNA
SEQ ID NO: 14: synthetic DNA
SEQ ID NO: 15: synthetic peptide
SEQ ID NO: 16: synthetic DNA
SEQ ID NO: 17: synthetic DNA
SEQ ID NO: 18: synthetic DNA
SEQ ID NO: 19: synthetic DNA
SEQ ID NO: 20: synthetic DNA
SEQ ID NO: 21: synthetic DNA
SEQ ID NO: 22: synthetic DNA
SEQ ID NO: 23: synthetic DNA
SEQ ID NO: 24: synthetic DNA
SEQ ID NO: 25: synthetic DNA

## Claims

1. A reagent for tumor detection, which comprises a probe for the FEAT gene or amplification primers for the FEAT gene, or an antibody against the FEAT protein or a fragment of the antibody.

2. A pharmaceutical composition for tumor prevention, which comprises a substance shown in (a) or (c) below or cells shown in (b) below:
(a) a substance capable of inhibiting FEAT gene expression or FEAT protein activity;
(b) immune cells capable of reacting with the FEAT protein; or
(c) a substance capable of binding to the FEAT protein, which substance carries an antitumor agent, the substance shown in (a) above or the cells shown in (b) above.

3. The pharmaceutical composition according to claim 2, wherein the substance capable of inhibiting FEAT gene expression is siRNA against the FEAT gene.

4. The pharmaceutical composition according to claim 2, wherein the substance capable of inhibiting FEAT protein activity is an antibody against the FEAT protein.

5. The pharmaceutical composition according to claim 2, wherein the immune cells are T cells, B cells or dendritic cells.

6. A method for tumor detection, which comprises reacting a test sample taken from a living body with the reagent according to claim 1.

7. The method according to claim 6, wherein the test sample is blood, a body fluid or a tissue section.

8. A tumor animal model, which consists of a transgenic non-human animal transformed with the FEAT gene.


### Sequence Listing

SEQUENCE LISTING

<110> KYUSHU UNIVERSITY

<120> A DETECTION AGENT AND PREVENTIVE PHARMACEUTICAL COMPOSITION OF
      TUMOUR

<130> PCT11-0038

<150> JP 2010-181835
<151> 2010-08-16

<160> 25

<170> PatentIn version 3.4

<210> 1
<211> 2100
<212> DNA
<213> Homo sapiens

<220>
<221> CDS
<222> (1)..(2100)

<400> 1
atg aac ctc tta cct aaa agt tcc agg gag ttt ggc tcc gtt gac tat      48
Met Asn Leu Leu Pro Lys Ser Ser Arg Glu Phe Gly Ser Val Asp Tyr
1               5                   10                  15

tgg gag aag ttc ttc cag cag cga gga aag aaa gct ttc gag tgg tat      96
Trp Glu Lys Phe Phe Gln Gln Arg Gly Lys Lys Ala Phe Glu Trp Tyr
            20                  25                  30

gga acc tac ctg gaa ctg tgc ggg gtg cta cat aaa tat atc aag ccc      144
Gly Thr Tyr Leu Glu Leu Cys Gly Val Leu His Lys Tyr Ile Lys Pro
        35                  40                  45

agg gaa aag gtg ctg gtg att ggg tgt ggc aac tca gaa ctg agt gag 192
Arg Glu Lys Val Leu Val Ile Gly Cys Gly Asn Ser Glu Leu Ser Glu
    50                  55                  60

caa ctg tat gat gtg ggc tat cgg gat ata gtg aac atc gac atc agt 240
Gln Leu Tyr Asp Val Gly Tyr Arg Asp Ile Val Asn Ile Asp Ile Ser
65                  70                  75                  80

gag gtt gtc atc aag caa atg aag gaa tgt aat gcc acc cga cgg ccc 288
Glu Val Val Ile Lys Gln Met Lys Glu Cys Asn Ala Thr Arg Arg Pro
                85                  90                  95

cag atg agc ttc ttg aag atg gac atg acg cag atg gag ttt cct gat 336
Gln Met Ser Phe Leu Lys Met Asp Met Thr Gln Met Glu Phe Pro Asp
            100                 105                 110

gcc tcg ttc cag gtg gtg ttg gac aag ggc acc ctg gat gct gtc ctg 384
Ala Ser Phe Gln Val Val Leu Asp Lys Gly Thr Leu Asp Ala Val Leu
        115                 120                 125

aca gat gag gaa gag aag acc tta caa cag gtg gac agg atg ctg gct 432
Thr Asp Glu Glu Glu Lys Thr Leu Gln Gln Val Asp Arg Met Leu Ala
    130                 135                 140

gag gtt ggc cgt gtc ctg cag gtg ggc ggt cgc tat ctc tgc atc tcc 480
Glu Val Gly Arg Val Leu Gln Val Gly Gly Arg Tyr Leu Cys Ile Ser
145                 150                 155                 160

ctg gct cag gct cac atc ctg aag aaa gca gtg ggc cac ttc tcc cgg 528
Leu Ala Gln Ala His Ile Leu Lys Lys Ala Val Gly His Phe Ser Arg
                165                 170                 175

gag ggg tgg atg gtg agg gtg cac caa gtg gcc aac agc cag gac cag 576
Glu Gly Trp Met Val Arg Val His Gln Val Ala Asn Ser Gln Asp Gln
            180                 185                 190

gtg ttg gaa gca gag cct cag ttc tcc ttg cct gtc ttt gcc ttc atc 624

Val Leu Glu Ala Glu Pro Gln Phe Ser Leu Pro Val Phe Ala Phe Ile
        195                 200                 205

atg acc aag ttc agg cca gtc cct ggc tct gcc ctt cag atc ttt gag     672
Met Thr Lys Phe Arg Pro Val Pro Gly Ser Ala Leu Gln Ile Phe Glu
    210                 215                 220

ctg tgt gct cag gag cag cgc aag cct gtg cgg ctg gag agt gcc gag     720
Leu Cys Ala Gln Glu Gln Arg Lys Pro Val Arg Leu Glu Ser Ala Glu
225                 230                 235                 240

cgg ctg gcc gag gcg gtg cag gag cga cag cag tat gcc tgg ctg tgc     768
Arg Leu Ala Glu Ala Val Gln Glu Arg Gln Gln Tyr Ala Trp Leu Cys
                245                 250                 255

agc cag ctg cgc cgc aag gcc agg ctg ggg agt gtg tct ctg gac ttg     816
Ser Gln Leu Arg Arg Lys Ala Arg Leu Gly Ser Val Ser Leu Asp Leu
            260                 265                 270

tgc gat ggg gac acg ggg gag cca cgc tac acc ctc cac gtg gtg gac     864
Cys Asp Gly Asp Thr Gly Glu Pro Arg Tyr Thr Leu His Val Val Asp
        275                 280                 285

agc ccc act gtg aaa cca tcg cgg gac aat cat ttt gcg att ttc atc     912
Ser Pro Thr Val Lys Pro Ser Arg Asp Asn His Phe Ala Ile Phe Ile
    290                 295                 300

atc cct cag ggc cgg gag acc gag tgg ctc ttt ggc atg gat gag ggc     960
Ile Pro Gln Gly Arg Glu Thr Glu Trp Leu Phe Gly Met Asp Glu Gly
305                 310                 315                 320

cgg aaa cag ctg gcg gcc agt gct ggc ttc agg agg ttg att aca gtg     1008
Arg Lys Gln Leu Ala Ala Ser Ala Gly Phe Arg Arg Leu Ile Thr Val
                325                 330                 335

gcc ctt cac cga ggt cag cag tat gaa agc atg gac cac atc caa gct     1056
Ala Leu His Arg Gly Gln Gln Tyr Glu Ser Met Asp His Ile Gln Ala
            340                 345                 350

gag ctg tcg gct aga gtc atg gag ctg gcc cca gct ggg atg ccc acc 1104
Glu Leu Ser Ala Arg Val Met Glu Leu Ala Pro Ala Gly Met Pro Thr
        355                 360                 365

cag cag cag gtc ccc ttt ctg tct gtg ggt ggg gac att ggg gtc cgg 1152
Gln Gln Gln Val Pro Phe Leu Ser Val Gly Gly Asp Ile Gly Val Arg
    370                 375                 380

acc gtt cag cac caa gac tgc agc ccc ttg agc ggt gac tat gtc att 1200
Thr Val Gln His Gln Asp Cys Ser Pro Leu Ser Gly Asp Tyr Val Ile
385                 390                 395                 400

gag gat gtg caa ggg gat gac aag cga tac ttc cgt cga ctg atc ttc 1248
Glu Asp Val Gln Gly Asp Asp Lys Arg Tyr Phe Arg Arg Leu Ile Phe
                405                 410                 415

ctc agc aac agg aat gtg gtg cag tcc gaa gcc agg ttg ctg aag gat 1296
Leu Ser Asn Arg Asn Val Val Gln Ser Glu Ala Arg Leu Leu Lys Asp
            420                 425                 430

gtg tct cac aaa gcc cag aag aag cgg aaa aag gac agg aag aag cag 1344
Val Ser His Lys Ala Gln Lys Lys Arg Lys Lys Asp Arg Lys Lys Gln
        435                 440                 445

cgg cct gct gat gcg gag gac ctc cct gca gcc ccg ggg cag tcc att 1392
Arg Pro Ala Asp Ala Glu Asp Leu Pro Ala Ala Pro Gly Gln Ser Ile
    450                 455                 460

gat aag agt tac ctg tgt tgt gaa cac cac aaa gcc atg atc gct ggc 1440
Asp Lys Ser Tyr Leu Cys Cys Glu His His Lys Ala Met Ile Ala Gly
465                 470                 475                 480

ctt gcc ctg ctg aga aac cca gag cta ctc cta gag atc cca ctg gca 1488
Leu Ala Leu Leu Arg Asn Pro Glu Leu Leu Leu Glu Ile Pro Leu Ala
                485                 490                 495

ttg ttg gtg gta ggc ctg ggc ggg ggc agc ctc ccc ctc ttt gtc cac 1536

Leu Leu Val Val Gly Leu Gly Gly Gly Ser Leu Pro Leu Phe Val His
            500                 505                 510

gat cat ttt cca aag tcc tgc att gat gct gtg gag atc gat ccc tcc 1584
Asp His Phe Pro Lys Ser Cys Ile Asp Ala Val Glu Ile Asp Pro Ser
        515                 520                 525

atg ttg gaa gtg gcc acc cag tgg ttt ggc ttc tcc cag agt gac cga 1632
Met Leu Glu Val Ala Thr Gln Trp Phe Gly Phe Ser Gln Ser Asp Arg
    530                 535                 540

atg aag gtc cac att gca gat ggc ctg gac tat atc gcc agc ttg gca 1680
Met Lys Val His Ile Ala Asp Gly Leu Asp Tyr Ile Ala Ser Leu Ala
545                 550                 555                 560

gga gga gga gaa gca cgg cct tgc tac gat gtc ata atg ttt gat gtt 1728
Gly Gly Gly Glu Ala Arg Pro Cys Tyr Asp Val Ile Met Phe Asp Val
                565                 570                 575

gac agt aag gac cca aca ctg gga atg agt tgt ccg ccc cca gca ttt 1776
Asp Ser Lys Asp Pro Thr Leu Gly Met Ser Cys Pro Pro Pro Ala Phe
            580                 585                 590

gtg gag caa tct ttt cta cag aag gtt aaa agc atc ttg act cct gaa 1824
Val Glu Gln Ser Phe Leu Gln Lys Val Lys Ser Ile Leu Thr Pro Glu
        595                 600                 605

ggt gtt ttt att ctc aac ctt gtg tgc cga gac ttg ggg cta aaa gac 1872
Gly Val Phe Ile Leu Asn Leu Val Cys Arg Asp Leu Gly Leu Lys Asp
    610                 615                 620

tca gtg ctg gct ggg ctc aag gca gtg ttc ccc ctc cta tat gtc cgg 1920
Ser Val Leu Ala Gly Leu Lys Ala Val Phe Pro Leu Leu Tyr Val Arg
625                 630                 635                 640

cga att gag ggt gaa gtg aat gag atc ctg ttc tgt cag ctg cac cct 1968
Arg Ile Glu Gly Glu Val Asn Glu Ile Leu Phe Cys Gln Leu His Pro
                645                 650                 655

gag caa aaa ctt gcc aca cca gag ctc cta gaa aca gcc cag gct ttg     2016
Glu Gln Lys Leu Ala Thr Pro Glu Leu Leu Glu Thr Ala Gln Ala Leu
            660                 665                 670

gag cgg acc ctg agg aag cct ggg agg ggt tgg gat gac acg tat gtc     2064
Glu Arg Thr Leu Arg Lys Pro Gly Arg Gly Trp Asp Asp Thr Tyr Val
        675                 680                 685

ttg tca gat atg ctc aag acg gtg aaa att gtg tga                     2100
Leu Ser Asp Met Leu Lys Thr Val Lys Ile Val
    690                 695

<210> 2
<211> 699
<212> PRT
<213> Homo sapiens

<400> 2

Met Asn Leu Leu Pro Lys Ser Ser Arg Glu Phe Gly Ser Val Asp Tyr
1               5                   10                  15

Trp Glu Lys Phe Phe Gln Gln Arg Gly Lys Lys Ala Phe Glu Trp Tyr
            20                  25                  30

Gly Thr Tyr Leu Glu Leu Cys Gly Val Leu His Lys Tyr Ile Lys Pro
        35                  40                  45

Arg Glu Lys Val Leu Val Ile Gly Cys Gly Asn Ser Glu Leu Ser Glu
    50                  55                  60

Gln Leu Tyr Asp Val Gly Tyr Arg Asp Ile Val Asn Ile Asp Ile Ser

65                  70                  75                  80

Glu Val Val Ile Lys Gln Met Lys Glu Cys Asn Ala Thr Arg Arg Pro
                85                  90                  95

Gln Met Ser Phe Leu Lys Met Asp Met Thr Gln Met Glu Phe Pro Asp
            100                 105                 110

Ala Ser Phe Gln Val Val Leu Asp Lys Gly Thr Leu Asp Ala Val Leu
        115                 120                 125

Thr Asp Glu Glu Glu Lys Thr Leu Gln Gln Val Asp Arg Met Leu Ala
    130                 135                 140

Glu Val Gly Arg Val Leu Gln Val Gly Gly Arg Tyr Leu Cys Ile Ser
145                 150                 155                 160

Leu Ala Gln Ala His Ile Leu Lys Lys Ala Val Gly His Phe Ser Arg
                165                 170                 175

Glu Gly Trp Met Val Arg Val His Gln Val Ala Asn Ser Gln Asp Gln
            180                 185                 190

Val Leu Glu Ala Glu Pro Gln Phe Ser Leu Pro Val Phe Ala Phe Ile
        195                 200                 205

Met Thr Lys Phe Arg Pro Val Pro Gly Ser Ala Leu Gln Ile Phe Glu
    210                 215                 220

Leu Cys Ala Gln Glu Gln Arg Lys Pro Val Arg Leu Glu Ser Ala Glu
225                 230                 235                 240

Arg Leu Ala Glu Ala Val Gln Glu Arg Gln Gln Tyr Ala Trp Leu Cys
                245                 250                 255

Ser Gln Leu Arg Arg Lys Ala Arg Leu Gly Ser Val Ser Leu Asp Leu
            260                 265                 270

Cys Asp Gly Asp Thr Gly Glu Pro Arg Tyr Thr Leu His Val Val Asp
        275                 280                 285

Ser Pro Thr Val Lys Pro Ser Arg Asp Asn His Phe Ala Ile Phe Ile
    290                 295                 300

Ile Pro Gln Gly Arg Glu Thr Glu Trp Leu Phe Gly Met Asp Glu Gly
305                 310                 315                 320

Arg Lys Gln Leu Ala Ala Ser Ala Gly Phe Arg Arg Leu Ile Thr Val
                325                 330                 335

Ala Leu His Arg Gly Gln Gln Tyr Glu Ser Met Asp His Ile Gln Ala
            340                 345                 350

Glu Leu Ser Ala Arg Val Met Glu Leu Ala Pro Ala Gly Met Pro Thr
        355                 360                 365

Gln Gln Gln Val Pro Phe Leu Ser Val Gly Gly Asp Ile Gly Val Arg

    370                 375                 380

Thr Val Gln His Gln Asp Cys Ser Pro Leu Ser Gly Asp Tyr Val Ile
385                 390                 395                 400

Glu Asp Val Gln Gly Asp Asp Lys Arg Tyr Phe Arg Arg Leu Ile Phe
                405                 410                 415

Leu Ser Asn Arg Asn Val Val Gln Ser Glu Ala Arg Leu Leu Lys Asp
            420                 425                 430

Val Ser His Lys Ala Gln Lys Lys Arg Lys Lys Asp Arg Lys Lys Gln
        435                 440                 445

Arg Pro Ala Asp Ala Glu Asp Leu Pro Ala Ala Pro Gly Gln Ser Ile
    450                 455                 460

Asp Lys Ser Tyr Leu Cys Cys Glu His His Lys Ala Met Ile Ala Gly
465                 470                 475                 480

Leu Ala Leu Leu Arg Asn Pro Glu Leu Leu Leu Glu Ile Pro Leu Ala
                485                 490                 495

Leu Leu Val Val Gly Leu Gly Gly Gly Ser Leu Pro Leu Phe Val His
            500                 505                 510

Asp His Phe Pro Lys Ser Cys Ile Asp Ala Val Glu Ile Asp Pro Ser
        515                 520                 525

Met Leu Glu Val Ala Thr Gln Trp Phe Gly Phe Ser Gln Ser Asp Arg
    530                 535                 540
Met Lys Val His Ile Ala Asp Gly Leu Asp Tyr Ile Ala Ser Leu Ala
545                 550                 555                 560
Gly Gly Gly Glu Ala Arg Pro Cys Tyr Asp Val Ile Met Phe Asp Val
                565                 570                 575
Asp Ser Lys Asp Pro Thr Leu Gly Met Ser Cys Pro Pro Pro Ala Phe
            580                 585                 590
Val Glu Gln Ser Phe Leu Gln Lys Val Lys Ser Ile Leu Thr Pro Glu
        595                 600                 605
Gly Val Phe Ile Leu Asn Leu Val Cys Arg Asp Leu Gly Leu Lys Asp
    610                 615                 620
Ser Val Leu Ala Gly Leu Lys Ala Val Phe Pro Leu Leu Tyr Val Arg
625                 630                 635                 640
Arg Ile Glu Gly Glu Val Asn Glu Ile Leu Phe Cys Gln Leu His Pro
                645                 650                 655
Glu Gln Lys Leu Ala Thr Pro Glu Leu Leu Glu Thr Ala Gln Ala Leu
            660                 665                 670
Glu Arg Thr Leu Arg Lys Pro Gly Arg Gly Trp Asp Asp Thr Tyr Val

        675                 680                 685

Leu Ser Asp Met Leu Lys Thr Val Lys Ile Val
    690                 695

<210> 3
<211> 19
<212> DNA
<213> Artificial

<220>
<223> Synthetic DNA

<400> 3
actgagtgag caactgtat 19

<210> 4
<211> 19
<212> DNA
<213> Artificial

<220>
<223> Synthetic DNA

<400> 4
atgaaggaat gtaatgcca 19

<210> 5
<211> 19
<212> DNA
<213> Artificial

<220>
<223> Synthetic DNA

<400> 5
agtccattga taagagtta 19

<210> 6
<211> 19
<212> DNA
<213> Artificial

<220>
<223> Synthetic DNA

<400> 6
agtcctgcat tgatgctgt 19

<210> 7
<211> 19
<212> DNA
<213> Artificial

<220>
<223> Synthetic DNA

<400> 7
tgaaggtcca cattgcaga 19

<210> 8
<211> 19
<212> DNA
<213> Artificial

<220>
<223> Synthetic DNA

<400> 8

acgtatgtct tgtcagata 19

<210> 9
<211> 33
<212> DNA
<213> Artificial

<220>
<223> Synthetic DNA

<400> 9
ggggtaccat gttgatttct tcacggatcg ttc 33

<210> 10
<211> 33
<212> DNA
<213> Artificial

<220>
<223> Synthetic DNA

<400> 10
gcgctcgagt taaactttaa tgccccaatg gat 33

<210> 11
<211> 20
<212> DNA
<213> Artificial

<220>
<223> Synthetic DNA

<400> 11
   aaagtgtgag gggctcttca 20

<210> 12
<211> 20
<212> DNA
<213> Artificial

<220>
<223> Synthetic DNA

<400> 12
ccctaatctt caggcaacct 20

<210> 13
<211> 21
<212> DNA
<213> Artificial

<220>
<223> Synthetic DNA

<400> 13
tcctgtggca tccacgaaac t 21

<210> 14
<211> 21
<212> DNA
<213> Artificial

<220>
<223> Synthetic DNA

<400> 14
gaagcatttg cggtggacga t 21

<210> 15

<211>  11
<212>  PRT
<213>  Artificial

<220>
<223>  Synthetic Peptide

<400>  15

Tyr Ala Arg Ala Ala Ala Arg Gln Ala Arg Ala
1               5                   10

<210>  16
<211>  20
<212>  DNA
<213>  Artificial

<220>
<223>  Synthetic DNA

<400>  16
ctagcattca ggccctcatc                                                    20

<210>  17
<211>  21
<212>  DNA
<213>  Artificial

<220>
<223>  Synthetic DNA

<400>  17
gaaaagtctg cctgtcttcc a                                                  21

<210>  18

<211>  20
<212>  DNA
<213>  Artificial

<220>
<223>  Synthetic DNA

<400>  18
gctcctcccc agcatcttat                                                    20


<210>  19
<211>  20
<212>  DNA
<213>  Artificial

<220>
<223>  Synthetic DNA

<400>  19
   ttgtgtctca gccctgaagt                                                   20


<210>  20
<211>  20
<212>  DNA
<213>  Artificial

<220>
<223>  Synthetic DNA

<400>  20
gctccgatgg tgatggtaag                                                    20


<210>  21
<211>  20
<212>  DNA

<213>  Artificial

<220>
<223>  Synthetic DNA

<400>  21
tagcactcag gagggtgagg                                                    20

<210>  22
<211>  18
<212>  DNA
<213>  Artificial

<220>
<223>  Synthetic DNA

<400>  22
cctgaagctc agcgcaca                                                      18

<210>  23
<211>  20
<212>  DNA
<213>  Artificial

<220>
<223>  Synthetic DNA

<400>  23
ccctcatcta gcgtctcagg                                                    20

<210>  24
<211>  20
<212>  DNA
<213>  Artificial

<220>
<223>  Synthetic DNA

<400>  24
tctccttggc tgcctttcta                                                    20

<210>  25
<211>  20
<212>  DNA
<213>  Artificial

<220>
<223>  Synthetic DNA

<400>  25
gtcacacagc cctgtcaaga                                                    20